(19) Europäisches Patentamt
European Patent Office
Office européen des brevets

(11) **EP 4 277 685 B1**

(12) **EUROPEAN PATENT SPECIFICATION**

(45) Date of publication and mention of the grant of the patent:
**19.08.2026 Bulletin 2026/34**

(21) Application number: **22739959.9**

(22) Date of filing: **12.01.2022**

(51) International Patent Classification (IPC):
***A61M 25/00*** *(2006.01)* ***A61M 25/14*** *(2006.01)*
***A61M 25/18*** *(2006.01)* ***A61M 99/00*** *(2012.01)*

(52) Cooperative Patent Classification (CPC):
(C-Sets available)
**A61M 13/003; A61B 5/4325; A61B 5/6852;**
A61B 2017/4216; A61K 9/0034; A61K 47/12;
A61M 31/00; A61M 2202/0225; A61M 2202/0468;
A61M 2205/0238; A61M 2205/123;
A61M 2205/3334; A61M 2205/3375; A61M 2205/75;
A61M 2210/1433 (Cont.)

(86) International application number:
**PCT/US2022/012081**

(87) International publication number:
**WO 2022/155174 (21.07.2022 Gazette 2022/29)**

(54) **SYSTEM FOR DELIVERING THERAPEUTIC AGENTS TO THE UTERINE CAVITY**

SYSTEM ZUR ABGABE VON THERAPEUTISCHEN MITTELN IN DIE GEBÄRMUTTERHÖHLE

SYSTÈME POUR ADMINISTRER DES AGENTS THÉRAPEUTIQUES DANS LA CAVITÉ UTÉRINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.01.2021 US 202163138556 P**

(43) Date of publication of application:
**22.11.2023 Bulletin 2023/47**

(73) Proprietor: **Gynion, LLC**
**Trumbull, CT 06611 (US)**

(72) Inventors:
- **SHIKHMAN, Oleg**
  **Trumbull, CT 06611 (US)**
- **TOMASHEVSKAIA, Marina**
  **Trumbull, CT 06611 (US)**

(74) Representative: **Lees, Kate Jane**
**Kate Lees Consulting Ltd t/a Sapphire IP**
**Offices 9-10 Hanley Workshops**
**Hanley Swan**
**Worcestershire WR8 0DX (GB)**

(56) References cited:
**WO-A1-2017/151918**
**US-A1- 2005 255 039**
**US-A1- 2007 112 409**
**US-A1- 2010 087 798**
**US-A1- 2017 258 511**
**US-A1- 2018 360 424**
**US-A1- 2020 261 707**
**US-A1- 2020 261 707**
**US-B1- 6 526 976**
**US-B2- 8 324 193**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
A61M 2202/0225, A61M 2202/0007;
A61M 2202/0468, A61M 2202/0007

## Description

1. Field of the description

This description relates to a system and method for delivering therapeutic agents to a patient and, more specifically, to delivering agents to a body cavity such as a uterine cavity for endometrial ablation.

2. Background

Heavy Menstrual Bleeding (HMB) is excessive bleeding from the vagina of over 80 mL of blood per period. Heavy periods can cause pain and discomfort and increase the risk of iron-deficiency anemia. Acute excessive bleeding can lead to hemodynamic instability, requiring hospitalization for fluid volume management, blood transfusion, and/or intravenous estrogen. This condition has a significant negative impact on woman's sexual functioning, mental well-being and overall health.

Studies have shown that Heavy Menstrual Bleeding affects approximately 1 in 3 women in their lifetime. This is over 200 million women worldwide. In the U.S. alone, there are ten million women suffering from HMB with 200,000 newly diagnosed women each year. The conservatively estimated annual direct economic cost of HMB in the US is approximately $1-1.55 billion and indirect cost is $12-36 billion.

There are four groups of treatment options that are currently available for treating HMB: 1) Dilatation and Curettage (D&C); 2) Hysterectomy; 3) Intrauterine device (IUD) and 4) Global endometrial ablation (GEA) devices. Each of these treatments has significant disadvantages. Dilation and Curettage offers a short-term relief and has a high risk of perforations. This option is not in wide use. Hysterectomy is a surgical removal of the uterus, which involves major surgery done under general anesthesia. Due to its invasive nature, high costs and risks, the number of these procedures has dropped over 50% in the last decade. Intrauterine devices, such as the Bayer HealthCare' "Mirena" IUD, are not highly effective and have significant hormonal side effects. Yet, use of the Mirena IUD to control heavy menstrual bleeding in women seeking contraception has increased in popularity due to ease-of-use and relatively low cost of this treatment option. Global Endometrial Ablation devices, such as the Hologic "NovaSure" and the Boston Scientific "Genesys HTA", are currently being utilized to ablate endometrium. The procedure can be done in a hospital setting or in the office. The procedure has demonstrated high efficacy, but is rather complex for in-office use and relatively expensive. Thus, GEA and IUD devices are the primary options for HMB treatment that are currently offered.

Endometrial ablation techniques, which have evolved as an alternative to hysterectomy, (e.g., laser, resecting loop with electric current, electric rollerball, thermal fluid-filled balloon, radiofrequency, freezing, heated saline) destroy some of the lining of the uterus in an attempt to control excessive bleeding. After endometrial ablation, pregnancy is not likely to occur.

The early techniques of endometrial ablation, introduced in the 1980s and still used today (although much less commonly) involve the use of a hysteroscope with either a "rollerball" or wire loop through which electrical heat travels to remove (resection) the endometrial lining. After the uterus is filled with fluid to enlarge it for better viewing, the surgeon moves the rollerball back and forth across the lining or uses the wire loop to shave off the tissue. Potential risks of this ablation method include infection, perforation of the uterus, cervical laceration, and fluid overload.

In 1997, the Food and Drug Administration (FDA) approved ThermaChoice, the first non-hysteroscopic ablation device to treat excessive uterine bleeding (menorrhagia) due to benign (non-cancerous) causes. The Gynecare ThermaChoice Uterine Balloon Therapy System has a balloon that is inserted through the neck of the cervix and into the uterus. Through a catheter connected to a controller console, the balloon is inflated with fluid and heated to 188°F (87°C) for 8 minutes to destroy the uterine lining.

In 2001, the FDA approved three more similar devices. These devices are to be used only in women who have not yet reached menopause and whose child-bearing is completed. The BEI Medical Systems Hydro ThermAblator delivers heated saline solution into the uterus. The heated saline solution is delivered using hysteroscopic guidance. The heated solution destroys the uterine lining in about ten minutes. The CryoGen Her Option Uterine Cryoblation Therapy System uses a cryoprobe capable of producing temperatures down to minus 148°F (minus 100°C) at the tip. This extreme cold is applied to the tissue for ten minutes to freeze and destroy the uterine lining. Ultrasound is used to guide and monitor the procedure.

Currently available GEA treatment options are expensive and complex. As a result, only 15.8% of patients received a therapeutic procedure within twelve months, post diagnosis. Studies also show that 38% of women with HMB undergo a hysterectomy, which is a major surgery, without even being offered less invasive alternatives. These results show that physicians and patients are well-aware of these limitations and reluctant to use these treatment options.

There is a need for a non-invasive, easy-to-use (short learning curve), and effective device for treating HMB. It would further be advantageous to provide such treatment with a low cost device and low procedural costs. This would enable treatment of the patient population that currently remains untreated due to clinical and economic limitations of the

current options. It would also be advantageous if such device ensured that the therapeutic agent is safely delivered to the endometrium in the uterine cavity.

Commonly assigned U.S. Patent No.10,485,962 and Publication Nos. 2019/0381294 and 2020/0261707 disclose various devices for delivery of therapeutic agents. Although these devices are effective in certain clinical applications, it would be advantageous to provide systems which better utilize aspiration and timing of agent delivery and removal and are more streamlined, as well as systems for delivery of therapeutic agents of increased viscosity for certain applications.

Furthermore, it is known in the art to apply therapeutic agents, such as trichloroacetic acid (TCA), to treat tissues in certain clinical procedures. However, the challenge with some of these devices/applicators which apply the agents is how to restrict undesired flow to healthy tissue while adequately targeting and penetrating the target tissue. Therapeutic agent compositions which achieve this would be clinically advantageous and broaden clinical applications of therapeutic agents. US 2005/255039 also discloses devices, methods and compositions known from the prior art.

## SUMMARY

The present teaching overcomes the deficiencies and disadvantages of the devices discussed above. The present teaching advantageously provides in preferred embodiments an apparatus for endometrial ablation that is easy to use, economical and controls the pressure of therapeutic agent applied to the endometrium. The apparatus of the present teaching also in preferred embodiments apply a pre-check of the uterine cavity to ensure it is sealed before application of the therapeutic agent, thereby preventing exposure to the agent in other areas of the body. The therapeutic agent is preferably injected to maximize the surface of exposure of the endometrium to the agent (preferably the entire surface of the endometrium will be exposed) to the agent while preventing leakage from the uterine cavity to other areas of the body.

In some embodiments, the injected agent for treatment has a high concentration of TCA to enable penetration of tissue to desired depths, e.g., greater than 1mm or even further beyond the superficial layer, and an increased viscosity (e.g., higher than water) to better control flow, e.g., prevent accidental leakage/spillage. This is achieved by balancing the proportion of TCA and viscosity increasing thickener as discussed in detail below. Such high viscosity/high TCA concentration compositions, with low pH, have clinical applications beyond endometrial ablation. Such varying clinical uses are discussed in detail below. Also discussed below are various formulations for the therapeutic compositions which enable delivery in a gel form, or alternatively in a syrup-like form or a honey/molasses form, for controlled flow and penetration below the superficial level for patient treatment. Various devices for delivering such viscous TCA are also discussed below.

In accordance with one aspect of the present teaching, a system for delivering an agent to a body cavity of a patient is provided comprising, a) a control handle having at least one actuator and a valve assembly preferably including multiple valves (flow control mechanisms); and b) a catheter assembly having an attachment member and a catheter. The attachment member is attached to the control handle and has at least one tube for delivering the agent into the catheter for delivery into the body cavity, and the agent is injected at increased pressure.

In accordance with another aspect of the present teaching, a system for delivering an agent to a body cavity of a patient is provided comprising the steps of a) a control handle having at least one actuator and a valve assembly; b) a catheter having a first lumen for injection/instillation of the agent; and c) a fluid line communicating with an agent containment member for injecting a pressurized gas into the containment member to inject the agent from the container through the first lumen in the catheter and into the body cavity and wherein negatively pressurized gas moves the agent from the body cavity back into the containment member. The catheter can include a second lumen for injection of $CO_2$ to check integrity of the body cavity before injection of the agent or alternatively the $CO_2$ can be injected through the first lumen.

In accordance with another aspect of the present teaching, a system for delivering an agent to a body cavity of a patient is provided comprising the steps of a) a control handle having a first actuator and a second actuator, the first actuator movable to open a first valve to enable injection of a pressurized gas to perform a cavity integrity check, and the second actuator movable to open a second valve to enable injection of the agent under increased pressure; and b) a catheter having a first lumen for injection of the agent into the body cavity. $CO_2$ to check integrity of the body cavity before injection of the agent can be injected through the first lumen or through an independent second lumen.

In accordance with another aspect of the present teaching, a system for delivering an agent to a body cavity of a patient is provided comprising the steps of a) a control handle having at least one actuator and a valve assembly; and b) a catheter assembly having a first lumen for passage of a pressurized agent, the first lumen having at least one opening and the agent having a viscosity greater than water and a pH less than 2. $CO_2$ to check integrity of the body cavity before injection of the agent can be injected through the first lumen or through an independent second lumen.

In accordance with another aspect of the present teaching, a system for delivering an agent to a body cavity of a patient is provided comprising the steps of a) a control handle having at least one actuator and a valve assembly; and b) a catheter assembly having a first lumen for passage of pressurized gas and a second lumen for passage of a pressurized

agent, the first lumen having at least one opening. Wherein the agent is viscous and the opening is sized such that the flow of the agent via the first lumen is limited.

In accordance with another aspect of the present teaching, a method for injecting a therapeutic agent into a body cavity of a patient is provided comprising the steps of a) providing a system with first, second, third and fourth valves; b) opening a first valve to enable flow of a gas into the cavity to check integrity of the cavity to determine if there is leakage from the cavity, while all other valves remaining closed; c) closing the first valve after the integrity check of the cavity; d) opening a second valve and or a third valve, wherein opening the second valve enables priming a fluid line of the system and opening the third valve enables flow of the therapeutic agent into the cavity during priming; and e) opening the fourth valve to remove the agent from the cavity.

In some embodiments, the first valve is reopened to facilitate aspiration of the agent from the cavity while the fourth valve is opened in step (e).

In some embodiments, the system further comprises a fifth valve downstream of the third valve for controlling flow of the therapeutic agent, wherein the fifth valve opens in conjunction with opening of the third valve.

In some embodiments, the system further comprises a venturi pump, wherein a valve for the venturi pump is open during injection of the agent and remains closed during the cavity integrity test.

In accordance with another aspect of the present teaching, a method for injecting a therapeutic agent into a body cavity of a patient is provided comprising the steps of a) moving an actuator to open a first valve to enable flow of a gas into the cavity to check integrity of the cavity to determine if there is leakage from the cavity, and second, third and fourth valves remaining closed while the first valve is open; b) closing the first valve after the integrity check of the cavity; c) opening the third valve for agent injection, the first and fourth valves remaining closed; d) closing at least the third valve to enable the agent for a period of time to penetrate tissue for treatment; e) after the period of time, opening the first and fourth valves to enable respectively flow of the pressurized gas through the catheter and aspiration of agent from the cavity, the second valve remaining closed. In some embodiments, the method includes opening the second valve for priming.

In some embodiments, the first valve remains closed in step (e).

In some embodiments, a fifth valve downstream of the third valve for closing flow of the agent through the catheter, the fifth valve closed when the third valve is open.

In accordance with another aspect of the present teaching, a method for treatment of tissue beyond a superficial level is provided comprising applying to tissue a composition containing a therapeutic agent, the composition including over 50% weight by weight of the therapeutic agent and having a thickening agent to provide a viscosity greater than a viscosity of water and having a pH less than 2, the therapeutic agent remaining on the tissue for a predetermined period of time to penetrate the tissue beyond a superficial layer.

In some embodiments, penetration is to a depth greater than about 1mm; in other embodiments, penetration is to a depth between about 1mm to about 10mm.

In some other embodiments, where the composition is applied for a short period of time, for example, less than 1 minute, penetration is to a depth less than about 1mm.

In some embodiments, the composition contains over 3% weight by weight of the thickener.

In accordance with another aspect of the present teaching, a method for ablation of tissue beyond a superficial level in the cervix and the cervical canal is provided comprising the steps of:

> sealing the cervical canal to block flow of a composition containing a therapeutic agent beyond the target tissue;
> distributing the composition over the target tissue in the cervix and the cervical canal, the composition containing at least 50% weight by weight of a trichloroacetic therapeutic agent, at least 3% weight by weight of a thickening agent to increase the viscosity of the agent, and a pH less than 2; and
> maintaining the therapeutic agent in contact with target tissue for a period of time to enable penetration of the therapeutic agent beyond the superficial level and beyond a depth of 1mm.

In some embodiments, the composition has a pH below zero. In some embodiments, the agent penetrates tissue to a depth greater than 1mm but less than 10mm.

In accordance with another aspect of the present teaching, a composition for ablation of tissue beyond a superficial level is provided comprising at least 50% weight by weight of trichloroacetic agent, at least 3% weight by weight of a thickening agent to increase the viscosity of the agent, and a pH less than 2 to provide a high concentration of viscous agent to penetrate the tissue beyond the superficial layer while controlling flow of the composition to target tissue during application.

In some embodiments, the composition has a pH less than or equal to zero.

It should be appreciated that in some embodiments, compositions comprising less than 50% weight by weight of trichloroacetic agent and less than 3% weight by weight are also contemplated for ablation of tissue beyond a superficial level. While such compositions are not as effective, deeper tissue penetration could be achieved by increasing treatment time and repeat applications. The invention is as defined in the appended claims.

## BRIEF DESCRIPTION OF THE DRAWINGS

So that those having ordinary skill in the art to which the subject teaching appertains will more readily understand how to make and use the apparatus disclosed herein, preferred embodiments thereof will be described in detail hereinbelow with reference to the drawings, wherein:

Figure 1 is a perspective view of an embodiment of the system of the present teaching for delivering a therapeutic agent into a body cavity having an injection module, a control handle and a catheter assembly including a catheter attachment and a catheter;
Figure 2 illustrates the detachable interface between the control handle and the catheter of Figure 1;
Figure 3 is a cross-sectional view of the distal end of the catheter of Figure 1 showing a portion of the $CO_2$ line external of the main lumen of the catheter;
Figure 4 is a schematic diagram of the system of Figure 1;
Figure 5 is a side view of an alternate embodiment of the catheter of the present teaching having the $CO_2$ line alongside the catheter, the catheter shown positioned within the uterine cavity;
Figure 6 is a side view in partial cross section of the syringe style cartridge and catheter of Figure 5;
Figure 7 is a schematic diagram of an alternate system of the present teaching;
Figure 8 is a perspective view of an alternate embodiment of the system of the present teaching for delivering a therapeutic agent into a body cavity having an injection module, a control handle and a catheter assembly;
Figure 9 is a perspective view of the control handle and catheter assembly of the system of Figure 8;
Figure 10 is a perspective of the control handle of Figure 9 with the top cover removed to show the valve assembly inside the control handle;
Figures 11A and 11B are perspective views of the valve assembly of Figure 10;
Figure 12 is a perspective view of the catheter attachment showing the lever and pin of the pinch valve, the pinch valve shown in the closed position;
Figure 13A is a perspective view of the catheter attachment and catheter of the system of Figure 8, and showing the agent containing vial mounted to the catheter attachment;
Figure 13B is a perspective view of the catheter attachment of Figure 13A with the top cover removed to illustrate the fluid lines contained therein;
Figure 13C is a side view of the catheter attachment of Figure 13A with the top cover removed to illustrate the fluid lines contained therein;
Figure 14 is a top view of the catheter attachment of Figure 13A with the top cover removed to show the fluid lines contained therein;
Figure 15 is a top view similar to Figure 14 but showing the control handle attached to the catheter attachment;
Figure 16 is a perspective view of the catheter shaft of the catheter of Figure 8 and showing the $CO_2$ line within the shaft;
Figure 17 illustrates the catheter of Figure 8 positioned in the uterine cavity;
Figure 17A is a side view of an alternate embodiment of the agent containing vial having volume markings.
Figure 18 is a side view of the injection module of the system of Figure 8;
Figure 19 is a side view of the injection module of Figure 18 with the side panel removed to illustrate one embodiment of the arrangement of the internal components;
Figure 20 is a schematic diagram of the system of Figure 8 having a venturi pump; and
Figure 21 is a schematic diagram of an alternate embodiment of the system of the present teaching having an external aspiration source instead of the venturi pump as in Figure 20;
Figure 22 is a side view of the uterine cavity having a lesion;
Figure 23 is a side view of a delivery system for treating the lesion of Figure 21;
Figure 24 is a view similar to Figure 22 showing the delivery system of Figure 23 inserted into the uterine cavity for delivery of the agent;
Figure 24A is a close up view of the area of detail of Figure 24;
Figure 25 is a side view of an alternate embodiment of a delivery system of the present teaching;
Figure 26 is a side view of an alternate embodiment of the delivery system of Figure 25; and
Figure 27 is a side view of an alternate embodiment of a delivery system of the present teaching for topical applications.

## DETAILED DESCRIPTION OF PREFERRED EMBODIMENTS

The present description provides a chemical global endometrium ablation system (device) for the treatment of Abnormal Uterine Bleeding (AUB). The system advantageously: 1) provides in preferred embodiments a cavity integrity

checking feature to ensure absence of perforations, that the fallopian tubes are closed and the uterine cavity is sealed prior to injection of the chemical agent; and 2) provides in preferred embodiments injection of the chemical ablation agent at a desired controlled pressure through the catheter for application of the agent to the endometrium. The therapeutic agent, e.g., chemical ablation agent, is preferably injected at a controlled pressure to maximize the surface of exposure of the endometrium (preferably the entire surface will be exposed) to the agent while preventing leakage to other areas. In the absence of perforations, and when a cervical canal is sealed by the device, the uterine cavity should be sealed as long as injection pressure will remain below the pressure level that is necessary for flow of fluids via fallopian tubes, which is typically above 60-70 mmHg even for low viscosity fluids. Therefore, there are two pressure limits: 1) the upper limit to prevent leakage and 2) the lower limit to assure maximum exposure.

The system of the present teaching enables injection of a viscous ablation agent which in some embodiments can be in a syrup-like and in other embodiments a honey/molasses-like and/or a gel form. The higher viscosity agent, in certain applications, provides better control of the agent within the cavity as compared to a more fluid like substance. Various viscous agents are described below with selected concentrations of agent and thickener to achieve the desirable balance of viscosity and sufficient flow and tissue penetration to achieve the clinical objectives.

The system of the present teaching provides a streamlined configuration for agent injection to reduce the number of fluid lines and/or vials/container for agent and waste. It also maximizes filling of the cavity for the procedure and evacuation of the cavity after the procedure. The components and fluid lines of the system that achieve this are discussed in detail below.

Additionally, in some embodiments, the system of the present teaching provides a disposable catheter system which contains a catheter and tube set which is detachably mounted to a reusable control module containing a valve assembly. This is also described in detail below.

Each of the foregoing features and aspects of the present teaching are described in detail below and can all be utilized in the systems of the present teaching or only some utilized, which would still be advantageous.

One cauterizing agent which can be used is an acid such as trichloroacetic acid (TCA). Derivatives of trichloroacetic such as bichloroacetic acid, and other substances such as silver nitrate, and derivatives of silver nitrate can also be utilized in certain embodiments. TCA is a chemical agent that denatures on contact with protein and causes chemical cauterization on contact with tissue, but does not spread beyond where it is directly applied. Additionally, instead of chemical agents, other therapeutic and diagnostic agents can be delivered, the devices/systems herein not being limited to chemical endometrial ablation as for example a specially formulated substance, such as a therapeutic agent in the form of a drug with a pharmaceutical formula that is specially formulated for this application can be utilized.

Additionally, although disclosed for use within the uterine cavity for endometrial ablation, the apparatus and systems disclosed herein are not so limited and can be used for treatment of other conditions and/or for treatment in other areas (cavities, lumens, etc.) of the body. The various agents/drugs described herein thus have utilization for other treatments/procedures including diagnostics.

As used herein, the term ’proximal’’ denotes the portion of the device closer to the user and the term "distal" denotes the portion of the device further from the user. Also, the terms "apparatus" and "device" are used herein interchangeably.

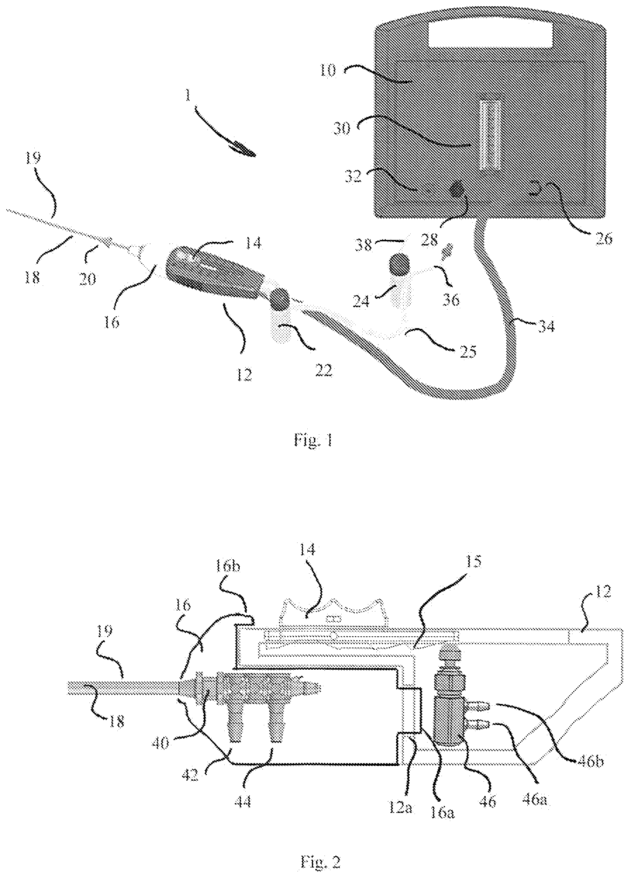

Turning now to the drawings wherein like reference numerals illustrate like parts throughout the several views, Figure 1 illustrates one embodiment of the system for delivery of a therapeutic agent into a body cavity, such as a uterine cavity.

The system 1 includes an injection/insufflation/instillation module 10, a control handle 12 and a catheter assembly comprising an attachment or adapter 16 and a catheter 18. The catheter attachment 16 is mounted/connected to the control handle 12. In some embodiments, the injection module 10 and the control handle 12 could be sterilizable and reusable, while the catheter attachment 16 and catheter 18 could be a single-use sterile device. Alternatively, the catheter attachment 16 and/or catheter 18 could be resterilizable and reusable. Yet another alternative is for the control handle 12 to be reusable, but instead of being sterile, it could be enclosed in a sterile protective cover that would create a sterile barrier.

The catheter attachment 16 provides for connection (attachment) of the catheter 18 to the control handle 12. The connection is preferably a removable connection so the catheter 18 and attachment 16 can be removed and either resterilized (in reusable embodiments) or discarded (in disposable embodiments). The catheter attachment 16 is detachably connected to a distal end of the control handle 12 and can be attached by a snap fit as shown, i.e., tab 16a engaging groove 12a and having a lip 16b overhanging an external surface of the control handle 12 see (Figure 2). Other types of detachable/removable connection, or in some embodiments, non-removable connection, are also contemplated. The catheter 18 and catheter attachment 16 can be the same piece or alternatively separate pieces. If separate pieces, in some embodiments, the catheter 18 is a separate component detachably mounted to the attachment component 16. Thus, the catheter 18 can be a separate component attachable to the catheter attachment 16 or alternatively the catheter 18 and catheter attachment 16 can be provided as an attached unit. Alternatively, the catheter 18 could be attached directly to the control handle 12.

The interface between the control handle 12 and catheter attachment 16 includes leak-proof connectors that

connect the flow lines of the control handle 12 and catheter 18 to each other so that fluid (liquid or gas) flow travels through tubes or lines into the control handle 12 and into lumens of catheter 18 as described in more detail below.

The injection module 10 has a flow meter 30 to regulate/monitor flow of fluid. Injection module further includes ports 26, 28 and 32 which are described below.

The control handle 12 is equipped with at least one actuator in the form of a slider 14 that activates liquid flow and gas flow by opening and closing various valves, for example trumpet valves and/or pinch valves. Alternatively, stopcock valves could also be used. Alternative actuators such as pivotable members instead of sliding mechanisms could be utilized to open the valve to allow fluid (gas or liquid) flow and to close the valve to block flow. The actuator(s) can have a normal position wherein the valve(s) is closed and the user actuates, e.g., moves, the actuator to close the valve. Alternatively, the actuator(s) can have a normal position wherein the valve(s) is open and the user actuates, e.g., moves, the actuator to close the valve.

Vial (agent storage container) 22 of the system stores the therapeutic agent, such as TCA, and is in fluid communication with the fluid line of the system for injecting the therapeutic agent at increased pressure. Vial (waste container) 24 of the system is utilized for collecting used therapeutic agent and other waste and bodily fluids at the end of the procedure which are aspirated from the body cavity into the container 24. Fluid line 25 communicates with waste container 24.

It should be appreciated that multiple sliders are contemplated in some embodiments. For example, one slider could control the valves in the integrity and aspiration subsystems, while the second slider could control the valves in the priming and injection subsystems and a third slider could control emptying of the cavity and/or evacuation of the unused therapeutic agent from the vial 22. In the embodiment of Figure 1, a single actuator 14 is provided.

The control handle 12 has a flow line 34 that connects to the injection module 10 via port 26. Flow line 34 extends into the control handle 12 and connects to a port to communicate via tubing within attachment 16 with a lumen in the catheter shaft 19 of catheter 18. Flow line 34 is a bundle of six fluid lines that connect the valves to the injection module 10. The control handle 12 also has an injection line 36 that connects to injection port 28 of the injection module 10 and an aspiration line 38 that connects to the aspiration port 32 of the injection module 10. Injection line 36 is in fluid communication with vial 22 via tube 25 which splits to provide a tube to communicate with vial 24. Injection line 36 extends into vial 22 via tube 25 to inject pressurized gas such as $CO_2$ to pressurize the agent contained in the vial 22. Aspiration line 38 is in fluid communication with vial 24 and extends into vial 24. Aspiration line 38 connects to a venturi pump 136 via port 32 of injection module 10 to create negative pressure in the vial 24 and aspirate the agent from the catheter 18.

Alternatively, the lines could be bundled, and the injection module 10 and the control handle 12 could be connected via a single port so that multiple connection ports for the integrity test, priming, aspiration and injection could be integrated into the control handle 12 at the point of interface with the catheter attachment 16. This is further described below.

The catheter attachment 16 includes a catheter 18 having a shaft 19 and a cervical plug 20. The cervical plug 20 of catheter 18 in some embodiments is slidable along the catheter shaft 19 to a desired position for plugging the cavity.

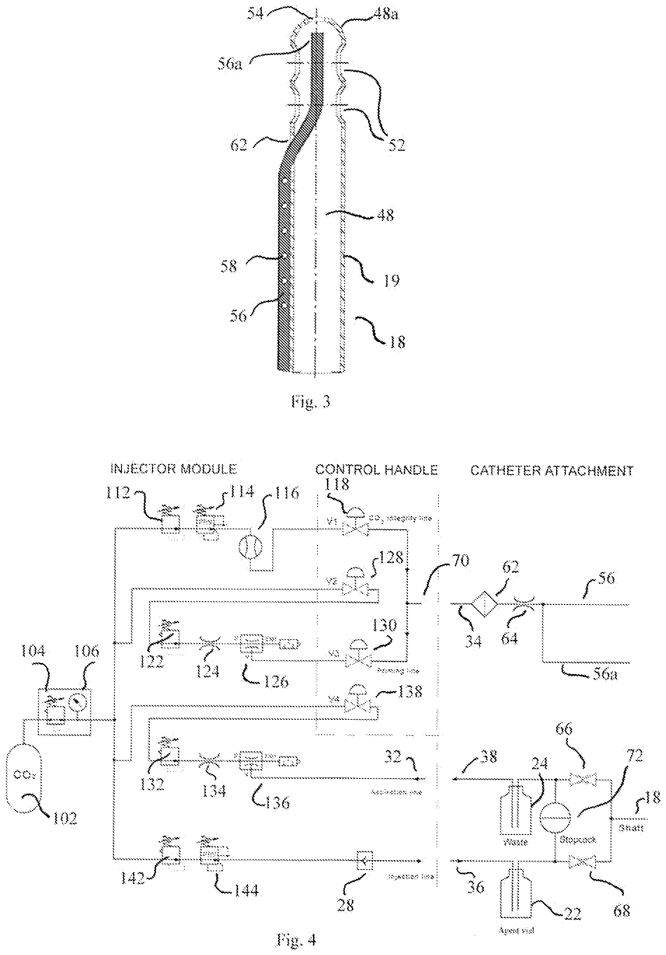

The detachable interface between the control handle 12 and the catheter attachment 16 is shown in Figure 2. The slide 14 has a cam surface 15 that controls opening and closing of the pinch valves (not shown) as it moves the pinch valve into engagement with the tube to compress the tube to a closed position. Cam surface 15 also engages the piston of trumpet valve 46 to depress the piston to actuate the valve. Multiple pinch valves and trumpet valves are contemplated, and only one is shown in Figure 2 by way of example. The trumpet valve 46 has ports 46a and 46b that connect to the $CO_2$ line 34 and control the flow of fluids through it. The shaft 19 of catheter 18 has a connector 40 that is attached to its proximal end. The connector 40 has a port 42 that is connected to the injection line 36 and a port 44 that is connected to the aspiration line 38. Ports 46a and 46b have respective input and output tubes. The other valves in the system (e.g., valves V1-V4 of Figure 4) also have input and output ports.

Figure 3 shows a distal portion of the shaft 19 of catheter 18. The shaft 19 has a main lumen 48 that is used for injection of therapeutic agent and aspiration of the body cavity. A smaller secondary lumen 56 is adjacent to the lumen 48 and used for the $CO_2$ inflow during the cavity integrity test and aspiration during priming. Priming facilitates evacuation of air and gases from the lines and the body cavity to avoid/eliminate/evacuate bubbles and air pockets. Lumen 56 runs alongside and external lumen 48 partially or for most of its length until a distal portion wherein it extends into main lumen 48 through side opening 62 in the wall of the catheter shaft 19.

The lumen 48 has at least one side opening 52 at a distal portion for the fluid flow in and out of the body cavity. Multiple openings 52 are also contemplated. The lumen 48 could also have alternatively or additionally a distal opening 54 aligned with the longitudinal axis of the catheter shaft 19. The opening 54 is located axially in the distal tip 48a of the lumen 48. In the illustrated embodiment, during the injection of the therapeutic agent, the agent flows distally via the internal channel of the lumen 48 and exits into the body cavity via the side openings 52 and end opening 54. During aspiration, the fluids from the body cavity reenter the internal channel of the lumen 48 via openings 52 and 54 and get evacuated into the waste container 24 as the fluids flow proximally within the lumen 48 and out through port 44 of connector 40 into the

attached tubing.

The lumen 56, extending alongside main lumen 48, has a distal opening in a distal tip 56a and, optionally, at least one side opening 58 for inflow of $CO_2$ into the during the cavity integrity test and outflow from the cavity of air and gasses during priming. A series of openings 58 are shown axially aligned. Multiple openings 58 spaced radially and/or axially could also be provided. When the catheter 18 is inserted into the uterine cavity the openings 58 are mostly located within the cavity. The opening(s) 58 in some embodiments are sized to minimize outflow of the therapeutic agent. (In such case, the opening 58 is smaller than the openings 52 and 54 for agent outflow). This sizing of the opening to prevent agent outflow can be achieved with a viscous therapeutic agent described in detail below.

The lumen 56 in the illustrated embodiment is angled (non-linear) at its distal end so that it extends from adjacent to the lumen 48 into the lumen 48. More specifically, in this angled lumen embodiment, a distal tip 56a of the lumen 56 extends within a distal region of lumen (internal channel) 48 as the lumen 56 extends through side opening 62 into lumen 48. This facilitates evacuation of air and gases from within the lumen 48 during priming, while the openings 58 evacuate air and gases from within the cavity. Additionally, this further enhances safety during the cavity integrity test. For example, if the distal tip 48a of the catheter 18 accidentally perforates the uterine wall, $CO_2$ that flows from the distal tip 56a of the lumen 56 will flow into the lumen 48 and leak via the opening 54 outside of the uterine cavity indicating that a perforation occurred. As illustrated, distal tip 56a terminates proximally of distal opening 54.

Thus, in the embodiment of Figure 3, catheter 18 has the lumen 56 that is mostly located outside of the main lumen 48 and a lumen 56a that is mostly located within the inner channel of the lumen 48. Such design could enhance the ability of the system to insufflate the cavity with $CO_2$ for the integrity test and evacuate air and gas during priming both from the cavity via the side opening 58 and from within the main lumen 48 via the distal opening in the lumen 56a.

Lumen 56 can be a separate tube attached to an external surface of the catheter shaft 19 which enters at a distal tip through a side opening, e.g., opening 62, of the catheter shaft 19 for positioning within lumen 48. Alternatively, the lumen 56 can be built into a wall of the shaft 19 or formed within a wall of the shaft 19 so as to be integral with the shaft 18.

Figure 4 is a schematic diagram of the system 1. The system 1 is powered by a $CO_2$ source 102, such as a disposable cartridge. The pressure from the source 102 is reduced with a primary pressure regulator 104. An optional pressure gauge 106 shows the output pressure. The $CO_2$ is then distributed into four subsystems: 1) integrity, 2) priming, 3) aspiration and 4) agent injection. Each subsystem has an individual/independent control of pressure and flow.

The integrity subsystem is used to test the integrity of the uterine cavity by insufflating it with $CO_2$ to detect its potential leakage via the fallopian tubes or an undiagnosed perforation. That is, the cavity integrity checking feature ensures absence of perforations, absence of leakage via fallopian tubes and that the cervical canal is sealed prior to injection of the chemical agent. The integrity subsystem has a pressure regulator 112 that controls/further reduces the $CO_2$ pressure. The pressure is preferably set/preset to a level that is known not to allow $CO_2$ to leak via fallopian tubes, which is typically below 60 mmHg. Other pressure settings are also contemplated. It also has a pressure relief valve 114 that is set to a pressure level slightly above the pressure setting of the regulator 112. If the pressure regulator 112 fails, the valve 114 will open automatically to relieve the excess pressure. The flow meter 116 indicates $CO_2$ flow through the integrity subsystem.

The lumen 56 of the catheter 18 connects to the integrity subsystem via the flow connector 70 which is one of the lines of bundle 34 (which can have four lines). The $CO_2$ flow from the integrity subsystem to the lumen 56 of the catheter attachment 16 is controlled by a normally-closed trumpet valve 118 (like valve 46). The catheter attachment 16 has a gas filter 62 therein (through which the $CO_2$ flows) to improve the cleanliness of the injected $CO_2$ and avoid patient cross-contamination and an optional orifice 64 to control the flow rate of the $CO_2$. For example, the flow rate could be set below 100 ml/min, which is a recommended limit for uterine insufflation. That is, one tube exits a port of the valve 118 and goes into the catheter attachment and connects to filter 62. (Flow connector 70 also connects to the output valve 130 to connect filter 62).

The priming subsystem is used for evacuation of air and gas from the lines and channels before and while the therapeutic agent is injected. The priming subsystem includes a pressure regulator 122 that regulates the $CO_2$ pressure input into a venturi pump 126. Alternatively, a vacuum pressure regulator could be placed after the pump 126, such that the venturi pump 126 could receive $CO_2$ directly from the primary regulator 104 while the vacuum pressure regulator could control the output pressure from the venturi pump 126. The vacuum pressure level, the size of the lumen 56 and the opening(s) 58 are preferably set to a level that is sufficient to evacuate air and gas, while minimizing outflow of the therapeutic agent. An optional orifice 124 could be used to limit a flow rate. Preferably, a normally-closed trumpet valve 128 controls $CO_2$ supply to the priming subsystem to avoid unnecessary leakage/loss of $CO_2$ due to venting of the venturi pump, while a trumpet valve 130 opens and closes priming aspiration flow from the lumen 56 of the catheter attachment 16.

The aspiration subsystem is used for creating a negative pressure/vacuum in the waste vial 24 and for evacuation of the therapeutic agent from the uterine cavity and the lines at the end of the treatment. The aspiration subsystem includes a pressure regulator 132 that regulates the $CO_2$ pressure input into a venturi pump 136. Alternatively, the venturi pump 136 could receive $CO_2$ directly from the primary regulator 104 while a vacuum pressure regulator placed after it could control the output pressure from the venturi pump 136. An optional orifice 134 could be used to limit a flow rate. Preferably, a normally-closed trumpet valve 138 controls $CO_2$ supply to the aspiration subsystem to avoid unnecessary leakage/loss of

$CO_2$ due to venting of the venturi pump. The aspiration subsystem connects to the line 38 via the port 32. A normally-closed pinch valve 66 controls the flow from the catheter 18 to the waste container 24.

The injection subsystem is used for injection of the therapeutic agent from the vial 22 into the cavity. The subsystem has a pressure regulator 142 that reduces the $CO_2$ pressure from the primary regulator 104. The pressure is preferably set/preset to a level that is equal or below the pressure level in the integrity subsystem. This ensures that the pressure utilized for the integrity check is not exceeded by the pressure utilized for injection/instillation of the therapeutic agent. A pressure relief valve 144 is set to a pressure level slightly above the pressure setting of the pressure regulator 142 so it will open to relieve excess pressure if the regulator 142 fails. The line 36 connects to the injection subsystem via a port 28, which could be a quick-connect port and/or a check valve. A normally-closed pinch valve 68 controls the flow from the vial 22 to the catheter 18.

The method of use will now be described with reference to the flow diagram of Figure 4.

During the system setup and preparation for use of this embodiment, all pinch and trumpet valves preferably remain closed. In alternate embodiments, one or more valves can have a normally open position and are moved to the closed position by the user for setup and preparation.

First, the integrity test of the cavity is initiated. During this test, the integrity test system (mode) is initiated by opening the trumpet valve 118 (V1), by moving an actuator such as actuator 14, and the uterine cavity is insufflated with $CO_2$ inflow from $CO_2$ source 102 as the gas flows through one of the flow lines of bundle 34 and through lumen 56 of catheter 18 exiting openings at the distal tip 56a and 58. When the pressure inside of the cavity equalizes with the pressure set by the regulator 112, the flow of $CO_2$ stops as indicated by the flow meter 116. The cessation can be automatic or alternatively or in addition by the user monitoring the pressure.

Upon completion of the integrity test, the trumpet valve 118 is closed, and the priming (step) mode is then initiated to purge $CO_2$ and air from the lines and the cavity.

The trumpet valves 128 (V2) and 130 (V3) are opened activating the priming subsystem (priming mode) that creates negative pressure in the lines and the cavity. Preferably, immediately after that, pinch valve 68 is also opened allowing the therapeutic agent to prefill the lines purging any remaining $CO_2$ and air out of these lines. Once all $CO_2$ and air are purged from the lines, the trumpet valves 128 and 130 are closed, while the pinch valve 68 remains open, so the injection subsystem fills the cavity with the therapeutic agent. Once the cavity is filled, the pinch valve 68 is reclosed to stop agent flow. Alternatively, the valves can remain open for the duration of the treatment keeping the therapeutic agent under pressure.

The therapeutic agent remains in the cavity for the duration of the treatment. At the end of the treatment, e.g., after a period of time which can be a preset time, the cavity and the lines are emptied by activating the aspiration system by opening the trumpet valve 138 and the pinch valve 66 while, preferably, simultaneously activating the trumpet valve 118 that reopens flow of $CO_2$. This allows the therapeutic agent to flow into the waste container 24, where it is optionally absorbed and/or neutralized by a special material. Once the flow from the cavity and the lines stops, the valves are closed.

Optionally, to evacuate the unused therapeutic agent directly from the vial 22, the stopcock 72 is opened, and the agent flows to the waste container 24 via flow line 25 under the pressure in the injection line. That is, when stopcock 72 is closed, suction pulls everything into the waste vial 24. When done, stopcock 72 is opened to allow suction to pull directly out of vial 22.

It should be appreciated, that instead of manual actuation to open and close the valves, e.g., via one or more manual actuators such as sliders with camming surfaces as in Figure 2, one or more of the valves for the four subsystems can be automatically activated via a programmed system. In such system, a control module would itself activate and deactivate one or more of the various subsystems via measuring/evaluation of various parameters and timing circuits. Alternatively, the user can activate one or more of the valves manually during the various steps (subsystems) of the procedure.

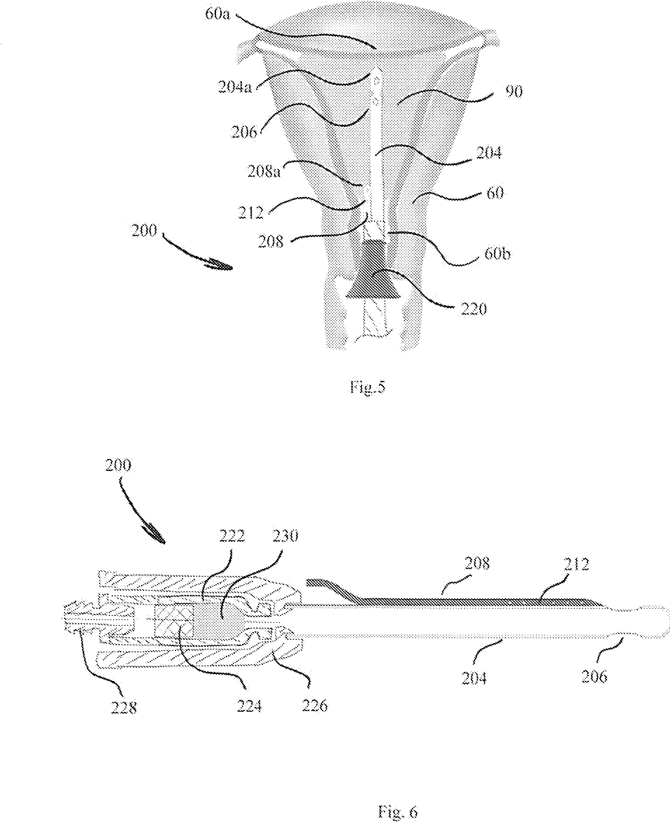

An alternative catheter design is illustrated in Figure 5. The catheter 200 is shown inserted into the uterine cavity of the uterus 60. The distal tip 204a of the lumen 204 of the catheter 200 is preferably positioned in the proximity to the fundus 60a of the uterus 60. The therapeutic agent 90 fills the uterine cavity after it is injected via the side openings 206 located near the distal tip 204a of the lumen 204. The cervical plug 220 is slid along the shaft to a position to close the cervical canal 60b to prevent leakage of fluids out of the uterine cavity. Lumen 208 is positioned alongside lumen 204 and remains outside of lumen 208 along its length. Lumen 208 can be a separate tube adjacent the catheter shaft containing the main lumen 204 or can be built into the catheter shaft. Lumen 208 is used for the $CO_2$ inflow during the cavity integrity test and aspiration during priming. The side openings 212 in the lumen 208 are sized such that air and gas can freely pass through them, while the fluid (agent), preferably a high viscosity fluid, cannot pass or pass at very low flow rate, so that it does not reach the connector 70. (That is, the side openings 212 are smaller than side openings 206). The high viscosity therapeutic agent 90 preferably begins filling the cavity from the fundal area 60a towards the cervical canal area 60b. During the agent injection, the air and gas e.g., $CO_2$, that might remain in the cavity and lines continue to flow out via the openings 212 as the agent fills the cavity and displaces the air and gas. But once the high viscosity therapeutic agent 90 fills the cavity, it covers and clogs the side openings 212 so that the outflow through openings 212 stops. Alternatively, the

outflow through openings 212 of the high viscosity therapeutic agent 90 could continue at a very slow rate. If that flow is monitored by a flow meter, this could serve as an indicator for when the cavity is filled with the agent.

In an alternative embodiment, the main lumen 204 is used for the $CO_2$ inflow during the cavity integrity test and the therapeutic agent 90 to fill the cavity, while the lumen 208 is used for aspiration of the air and gas during priming.

As shown in Figure 6, lumen 204 of catheter 200, with at least one opening 206, is mounted on a cartridge holder 226 that contains a syringe-style cartridge 222, which is preferably made of borosilicate glass. The plunger 224, which is preferably made from a Teflon-coated elastomer, moves axially within the cartridge 222 under $CO_2$ pressure. In some embodiments, the catheter 200 with the cartridge holder 226 is disposable and detachable from the control handle 12. In a preferred embodiment, the cartridge 222 is pre-filled with the agent. The pressure source is a line of the injection subsystem. In some embodiments, the cartridge holder 226 plugs directly into the control handle 12. In other embodiments, the line from the pressure source extends out of the handle 12, and the cartridge holder 226 is located closer to the distal end of the lumen 204 than to the handle 12. When the line is connected to the connector 228, and the injection subsystem is activated, $CO_2$ pressurizes the vial and pushes the plunger 224 distally to inject a therapeutic agent 230 into the lumen 204 at an increased pressure. Use of vacuum from the aspiration subsystem that would reverse/pull the plunger 224 proximally is also contemplated.

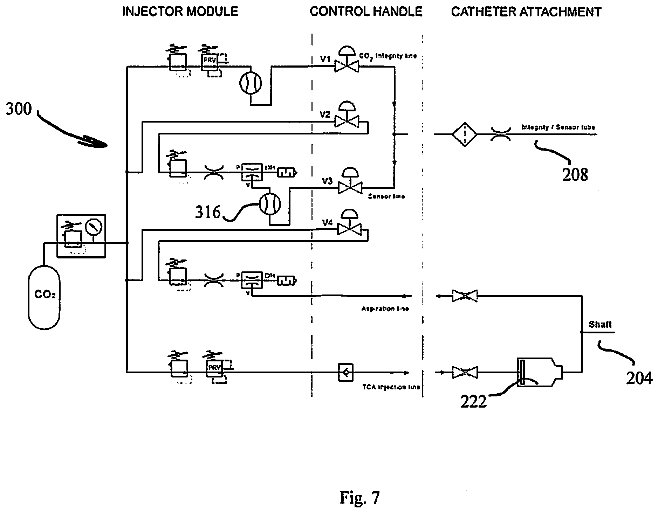

Fig. 7 is a schematic diagram of an alternative system of the present invention. This system 300 could be used with a therapeutic agent with a higher viscosity and with the catheter 200 that is described above and illustrated in Figures 5 and 6. The integrity subsystem of the system 300 operates in the identical fashion as in the system 1 of Figure 4. However, the priming subsystem in this embodiment of Figure 7 also serves as a sensor that indicates when the cavity is filled by the therapeutic agent. While air and gas flow through the lumen 208, the flow rate is high. But once the high viscosity therapeutic agent 90 fills the cavity, it covers and clogs the side openings 212, so that the flow through openings 212 stops or slows down significantly, the flow meter 316 will indicate that the cavity is full. Instead of a vial 22 that is used in the system 1, the system 300 uses an injection cartridge 222. As can be appreciated by a comparison of Figures 4 and 7, the regulators, valves, pumps, etc. of Figure 4 can be the same for the system of Figure 7 and thus in Figure 7 are not labeled except where they differ from Figure 4. In some embodiments, the ablation agent could remain in the cavity after the catheter is removed until it is absorbed/metabolized by the body. In other embodiments, a waste container like vial 24 for evacuation of the ablation agent at the end of the procedure can be incorporated into the system of Figure 7 as in the system of Figure 4.

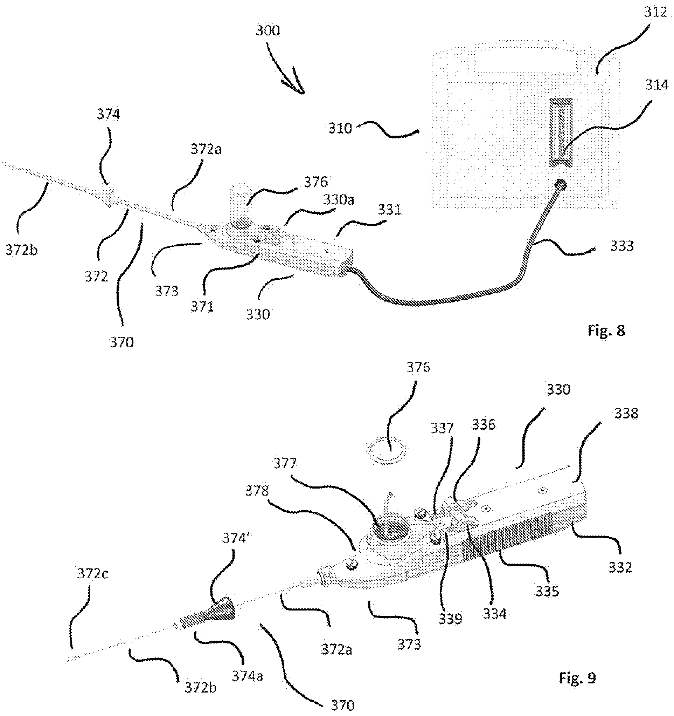

Figures 8-20 illustrate an alternative embodiment of the system of the present invention. In this embodiment, a single vial is utilized for a) storing the therapeutic agent prior to injection into the body cavity for treatment; and b) for storing the therapeutic agent when evacuated from the cavity after treatment. Further, this system differs from the system of Figure 1 in the mechanism and structure for opening and closing the valves for performing the cavity integrity check, priming, therapeutic agent injection and evacuation. Other differences will become apparent from the discussion below.

The system of Figures 8-19 provides a streamlined approach for applying a viscous therapeutic agent such as that described herein, although it can be used for injecting less or more viscous agents.

$CO_2$ is described herein as providing the pressurized fluid for the system, however, it should be appreciated that other fluids, e.g., pressurized gasses or liquids, can be utilized with the systems disclosed herein. Therefore, references to $CO_2$ herein are by way of example.

With initial reference to Figures 8 and 9, the system 300 includes an injection module 312, a control handle 330, a catheter attachment (adapter) 373 and a catheter 370. In the illustrated embodiment, the injection module 312 and control handle 330 are resterilizable and reusable and the catheter attachment 373 and catheter 370 are disposable. However, in alternate embodiments, the catheter 370 and/or catheter attachment 373 can be resterilizable and reusable and/or the control handle 330 can be disposable.

Control handle 330 is connected to the injection module 310 via a pneumatic line bundle 333. The bundle 333 includes multiple pneumatic lines/tubes that connect the pneumatic components of the injection module 310 to the pneumatic components of the control handle 330. A $CO_2$ module/storage unit containing $CO_2$ is provided within or adjacent the injection module 310 and is injected through the pneumatic line bundle 333 which communicates with the tubes within the control handle 330. The therapeutic agent is stored in vial 376 which is positioned in opening 377 in the catheter attachment 373. The vial 376 can be positioned in an inverted (upside down) position or in an upright position; in Figure 8 it is shown in the inverted position. The vial 376 is preferably only partially filled, e.g., one third or one half filled, with agent for reasons described below.

Control handle 330 has slidable actuators 334 and 336 which activate, i.e., open and close, the valves as described in detail below. The top cover of the control handle 330 is designated by reference numeral 338 and the bottom cover is designated by reference numeral 332 and either or both can have a knurled surface 335 for gripping by the clinician. A series of screws can be utilized to connect the covers as shown. Alternative attachment methods are also contemplated.

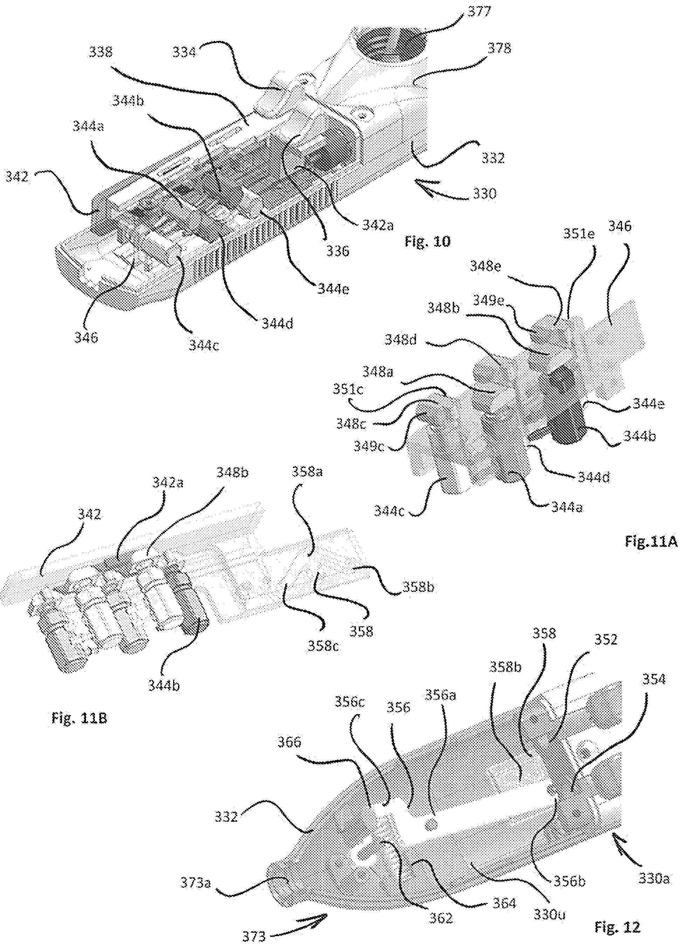

Catheter 370 is attached at a proximal portion to the distal portion of the catheter attachment 373 which is attached at a proximal portion to a distal portion of the control handle 330, with reference numeral 371 designating the

demarcation line between the control handle 330 and catheter attachment 373. Catheter 370 includes a catheter shaft 372 extending distally from distal nose portion 373a (Figure 12) of catheter attachment 373 and includes a cervical plug 374 which can be slid along the outer surface of the catheter shaft 372 to the desired position. Note Figures 8 and 9 show alternate embodiments of the cervical plug 374 wherein cervical plug 374 of Figure 8 has a conical shape and cervical plug 374' of Figure 9 has a conical shape and a distal region having a series of elastomeric ribs 374a which in certain instances can improve the seal within the cervical canal. The shaft 372 consequently can be considered as having a proximal shaft portion 372a proximal of the cervical plug 374 (or 374') and a distal shaft portion 372b distal of the plug 374 (or 374'). The length of the designated proximal and distal shaft portions 372a, 372b, respectively, will vary depending on the position of the cervical plug 374 (374') along the shaft 372.

In preferred embodiments, the outer diameter (OD) of proximal shaft portion 372a is greater than the OD of distal shaft portion 372b. The larger portion OD provides for a larger internal diameter (ID) to improve flow through the lumen of the catheter shaft 372. The reduced OD at shaft portion 372b provides a reduced profile for ease of insertion through the cervical canal which can reduce the need for cervical dilation. In alternate embodiments, the OD and/or ID of the shaft portions 372a and 372b are substantially the same.

The catheter 372 can be permanently fixed to the catheter attachment 373 or alternately removably connected to the catheter attachment 373. In some embodiments where removably attached, the catheter 370 can be disposable and the catheter attachment 373 reusable, or both could be disposable or both reusable.

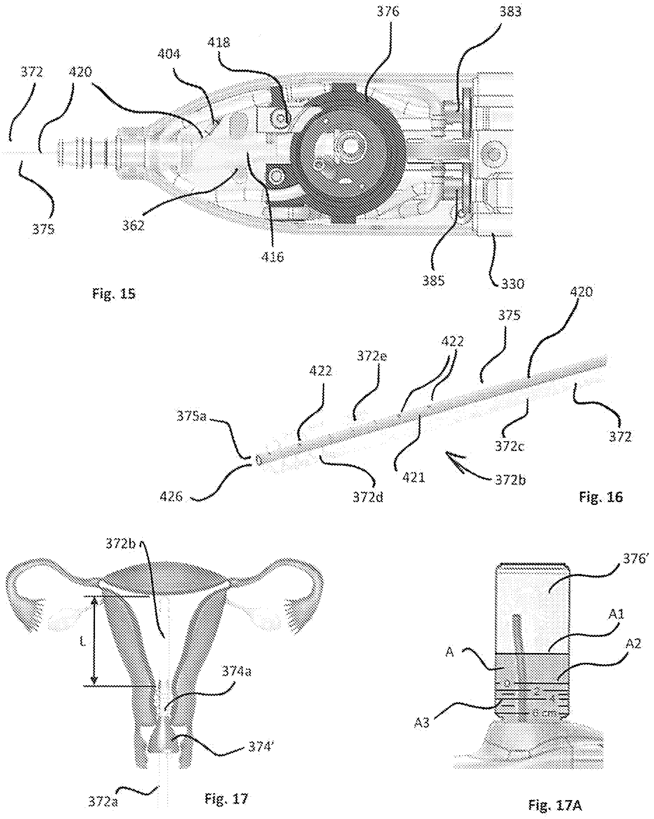

Catheter shaft 372, as shown in Figure 16, has an inner tube 420 forming an inner lumen 421 within the lumen 375 of the shaft 372. Inner tube 420 has a series of side holes 422 in its wall along a length, i.e., spaced apart axially. The inner tube 420 can also have a distal hole 426 aligned with the longitudinal axis. The inner tube 420 terminates in proximity of the distal end 372c of the catheter shaft 372. In some embodiments, the distal end of the inner tube 420 extends distally to the distal end 372c of the catheter shaft 372, while in other embodiments it terminates proximally to it. The number, size, location and shape of the holes can vary from that shown in Figure 16. The holes enable exit of $CO_2$ from line 333 which flows through the tubing in the catheter attachment 373 and into lumen 420. The lumen 375 of the catheter shaft forms an outer lumen. The catheter shaft 372 has spaced apart openings in its outer wall. The openings can be of various shapes such as oblong openings 372e and circular openings 372d, and the number, placement, size and shape of the openings can vary from that shown in Figure 16. These openings 372d and 372e allow the egress of the pressurized therapeutic agent which is injected by pressurized $CO_2$ out of vial 376 and into lumen 375. Lumen 375 can also have a distal opening 375a aligned with the longitudinal axis for egress of agent. The tube 420 can terminate proximally of the distal end of the lumen 375 as in the embodiment of Figure 3. Alternatively, it could be flush or terminate distally of the distal end of lumen 375. Openings 422 are preferably smaller than openings 372d and 372e to prevent the flow of agent through openings 422 as described below.

As, can be appreciated, when $CO_2$ is injected for the initial cavity integrity check, the $CO_2$ flows through tube 420, exiting openings 422 into the lumen 375 of shaft 372, and out openings 372d and 372e, and distal opening 375a, if provided, of the shaft 372 into the body cavity. $CO_2$ can also flow out distal opening 426, if provided. For injection of the agent, the agent flows through lumen 375 and out side openings 372d, 372e, and 375a, if provided, into the body cavity for treatment. During aspiration of the agent after the procedure, the aspirated agent flows back through openings 372d, 372e, and 375a, if provided, and into lumen 375 and back to the agent container, e.g., vial, 376. The agent is sufficiently viscous, and preferably does not flow into inner lumen 421 of tube 420 due to its small internal diameter and the small sized openings 422.

The catheter shaft 372 is shown positioned within the uterine cavity in Figure 17, with the conical plug 374' positioned against the cervix to close the cervical canal 60b to prevent leakage of fluids out of the uterine cavity.

As noted above, the control handle 330 contains the valve assembly, and the catheter attachment 373 containing the tubing set is positioned distal/downstream of the control handle 335. Referring now more specifically to the control handle 330 and with reference to Figures 9-12, the actuators and valves will be discussed. The control handle 330 has two actuators: a) actuator 336 movable to control the flow of the therapeutic agent; and b) actuator 334 movable to control the flow of $CO_2$. The actuators 334 and 336 open and close a series of valves for the various steps in the procedure. The actuators 334 and 336 are shown in the form of sliders with buttons or finger engaging portion which are connected to the sliders or integral therewith. They slide within respective slots 339, 337, however, other forms of actuators including rotatable actuators, levers, etc. could alternatively be provided. Moreover, although two actuators are utilized, it is also contemplated that a different number of actuators could be utilized to open and close the various valves to achieve the functions of the systems described herein.

Actuator 334 has two positions - a retracted (rearward or proximal) position and an advanced (forward or distal) position. Actuator 334 is shown in the retracted position in Figure 9. To inject $CO_2$, the actuator 334 is slid forward within slot 339. This opens the valve for $CO_2$ flow. Thus, in this embodiment, the valve is normally in the closed position. (Note in an alternate embodiment, the actuator 334 can be slid rearward to open the valve). In alternate embodiments, the valve is normally in the open position and the actuator is slid (forward or rearward, depending on the configuration) to close the valve. Thus, in these embodiments, the valve is normally in the open position. Note the valve is opened and closed by user

activation, but the $CO_2$ valve and/or one or more of the other valves described herein can be programmed to open and close automatically based on measured parameters or operational sequence.

Actuator 336 has three positions. A middle position, also referred to as a neutral position, a retracted (rearward or proximal) position and an advanced (forward or distal) position. Actuator 336 is shown in the middle (neutral) position in Figure 9. In the middle position, inflow of therapeutic agent is off; in the forward position the valve is open for injection of pressurized therapeutic agent from vial 376; and in the rearward position, the valve is open for pulling of therapeutic agent out of the body cavity into the vial 376 due to suction inside the vial 376. Note in an alternate embodiment, the actuator 336 can be slid rearward to open the valve for injection of therapeutic agent and slid forward to pull the therapeutic agent out of the body cavity. In alternate embodiments, the valves are normally in the open position and the actuator is moved to close the valve. Alternatively, the actuators could be moved laterally (side to side), rotated, or their position otherwise changed to open and close the valves.

The valve mechanism includes cam plates 342 and 338 and a series of valves 344a, 344b, 344c, 344d and 344e (collectively valves 334). Cam plate 342 is operatively connected to actuator 336 to control valves 344b, 344c, 344d and 344e and cam plate 338 is operatively connected to actuator 334 to control valve 344a. Can plate 342 also controls the pinch valve. Cam plates 338 and 342 move/slide relative to mounting plate 346 which is fixedly secured e.g., bolted, to control handle 330 (Figure 1) to hold the valves. Cam plate 342 includes an L-shaped region 342a which extends under cam plate 338 as shown in Figure 10. Each valve 344a-344e, has a cap 348a, 348b, 348c, 348d and 348e, respectively, having a front (distal) and rear (proximal) cam surface which is engaged by the cam plate 342 depending on its direction of movement. The front cam surfaces are designated by reference numerals 349a-349e and the rear cam surfaces are designated by reference numerals 351a-351e, however, for clarity not all cam surfaces 349 and 351 are labeled in the drawings. The valves function as follows: valve 344a controls flow of $CO_2$ of the cavity integrity check; valve 344b is for the priming line; valve 344c is for the aspiration line; valve 334d is for the venturi pump and valve 334e is for TCA injection.

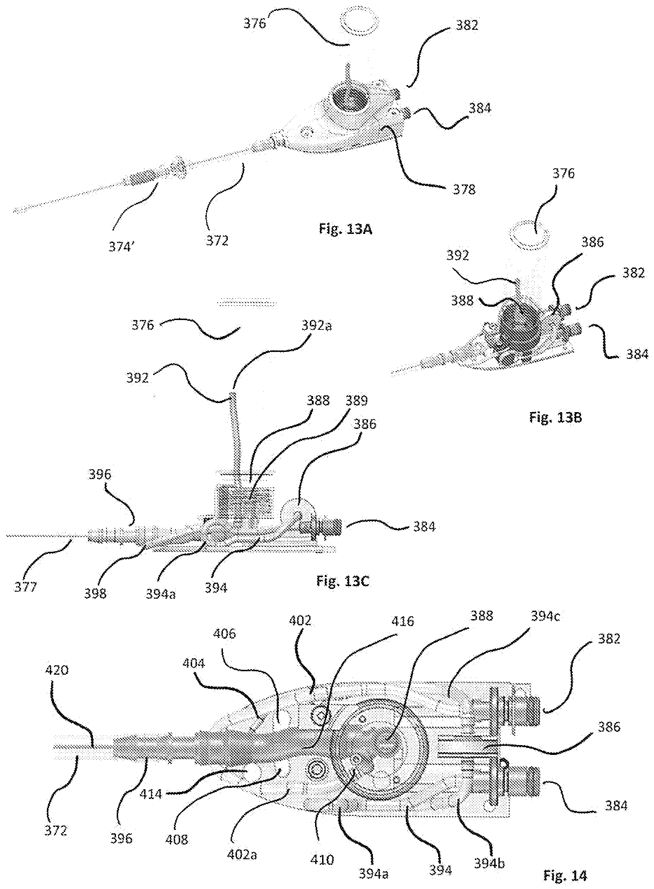

Actuator 336 is operatively connected to lever 356 within the catheter attachment 373 as shown in Figure 12. Thus, actuator 336, not only functions to open valves 344b-344e but to open and close a pinch valve downstream of the valves 344. The pinch valve includes a lever 356 which pivots about pivot pin 356a. Lever 356 has a U-shaped distal end 356c with a pin 362 extending transversely through a distal opening. Spring 364 biases lever 356 to the position of Figure 12 wherein the tube 416 for therapeutic agent injection is compressed. (Tube 416 is shown in Figures 14 and 15). Lever 356 includes proximal cam pin 356b that rides within V-slot of cam plate 342. As shown in Figure 11B, distal extension 358 of cam plate 342 has a V-slot 358 having a vertex 358a, distal leg 358b and proximal leg 358C. (Note that Figure 11B is in the opposite orientation of Figure 12 and the distal end of control handle 330 is designated by reference numeral 333). In the neutral position, cam pin 356b sits within vertex 358a of V-slot 358 and the pinch valve is closed. This neutral position is shown in Figure 12.

When actuator 336 is slid forward from its neutral position, the connected cam plate 342 is moved forward which moves V-slit 358 forward so that cam pin 356b engages/interacts with proximal leg 358c causing lever 356 to pivot about pivot point 356a so pin 362 moves away from post 366. This releases the compression/pinching of the tube 416 (not shown in Figure 12) between the post 366 and the pin 362 to open the pinch valve to enable fluid flow. When actuator 336 is moved rearward from its neutral position, cam plate 342 is moved rearward so that cam pin 356b engages/interacts with proximal leg 358b to pivot the lever 356 so that pin 362 moves away from post 366 to release a compression force on the elastomeric tube 416 to open the pinch valve to allow fluid flow. When actuator 336 is returned to the neutral position, pin 362 moves toward post 366 to apply a compressive force to the tube 416, thereby closing the pinch valve. Note post 366 is seated within slot 406 and pin 62 is seated within slot 408 of catheter attachment 373 (see Figure 14).

Additionally, when actuator 336 is slid forward or rearward from its neutral position, camming surfaces of cam plate 342 engage certain valves to open the valves as described herein.

In summary, with forward being distal movement and rearward being proximal movement actuation is as follows:

*actuator 334 movedfonvard → cam plate 338 moves forward → $CO_2$ valve opened.*
*actuator 334 moved reanvard → cam plate 338 moves rearward → $CO_2$ valve closed.*
*actuator 336 moved forward → cam plate 342 moves forward → pinch valve and priming, venturi and injection valves opened.*
*actuator 336 moved rearward → cam plate 342 moves backward → pinch valve and venturi and aspiration valves opened and $CO_2$ valve opened.*

Turning now to the catheter portion of the system, and with reference to Figures 13A-14, the catheter attachment 373 has a pneumatic connector 382 which engages connector 352 of control handle 330 and a pneumatic connector 384 which engages connector 354 of control handle 330 when the catheter attachment 373 is connected to the control handle 330. Opening 414 receives a screw for connecting the top and bottom housings of the catheter attachment 373. Note the catheter attachment 373 has a bottom portion which fits into or cooperates with the top (upper) portion 330u of the handle 330. In alternate embodiments, the top portion would instead be part of the catheter attachment 373 (and disposable with

attachment 373 in the disposable embodiments). In such embodiments, the mechanism for operating the pinch valve would be on the control handle 370 and the elastomeric or other portion for applying a pinching force to the tube would be in the catheter attachment 373, and when the catheter attachment 373 and control handle 370 are connected, the pinch valve would be mechanically connected for operation.

Extending into vial 376 is line (tube) 392 which provides a pressure line into the vial 376. Thus, $CO_2$ is pushed through the line 392 into the vial 376 to pressurize the vial 376 and push the pressurized therapeutic agent out of vial 376, through port 388, and into the main lumen 375 of catheter 372. Line 392 is also used for suction. That is, it applies a vacuum line to pull the agent though port 388 during the aspiration mode. This injection and suction are described in detail below. In preferred embodiments, the pressure line 392 has a distal opening 392a terminating above the fluid line of the agent in the vial 376. In some embodiments, the vial 376 can be about one third full with the agent (the rest would be filled with air). Thus, line 392 would be in communication with the air in the vial 376 above the fluid (agent) line; distal opening of port 388 would be in communication with the agent and thus within the fluid level.

In some embodiments, the vial 376 has an elastomeric stopper (plug), and the line 392 is a rigid spike preferably made from a metal hypotube with a sharp tip 392a that pierces through the stopper. In some embodiments, the line 392 has lateral openings for $CO_2$ or suction, preferably located proximally to the tip 392a, but above the fluid line of the agent in the vial 376.

The $CO_2$ flows through the connector 384 and through tube portion 394b of tube 394, around loop 394a, through filter 386, through tube portion 394c and then branches at connector 404 to extend through the elastomeric tube 416 and into the lumen 421 in internal tube 420 of catheter 370. Note the connector 404 pierces the wall of the elastomeric tube 416 to communicate with the interior of the tube 420 for passage of $CO_2$. The winding and looped shape of tube 394 maximizes the length of the tubing inside the catheter attachment 373 to create extra protection for reusable components. For example, if the agent gets into line 392 it won't travel back to the reusable control portion (control handle 330). Other arrangements for the tube 394 within the catheter attachment 372 are also contemplated. The filter for the $CO_2$ could be for example a .2 micron hydrophobic filter, or other filter.

With continued reference to Figure 14, tube 402 extends from connector 382, looping around the distal portion of the catheter attachment 373, and continuing at region 402a to connect to connector 410. Tube 392 is connected to connector 410 and extends into the vial 376. Elastomeric tube 416 is connected to port 388 which communicates with the vial 396. A barbed connector 396 connects at one end the elastomeric tube 416 and at the other end the catheter shaft 372. With the catheter shaft 372 fit over the barbed connector 396, agent flowing from the vial 376 through: port 388 and into elastomeric tube 416 continues through connector 396 and into lumen 375 of catheter shaft 372. For aspiration of the agent, fluid flow is in reverse-rearward through lumen 375 into elastomeric tube 416 via connector 396 and out through port 388 into vial 376. Note that the inner tube 420 of $CO_2$ extends through lumen 375 of shaft 372 and through connector 396 and communicates with tube connector 440 at a proximal region proximal of where the catheter shaft 372 is connected to connector 396.

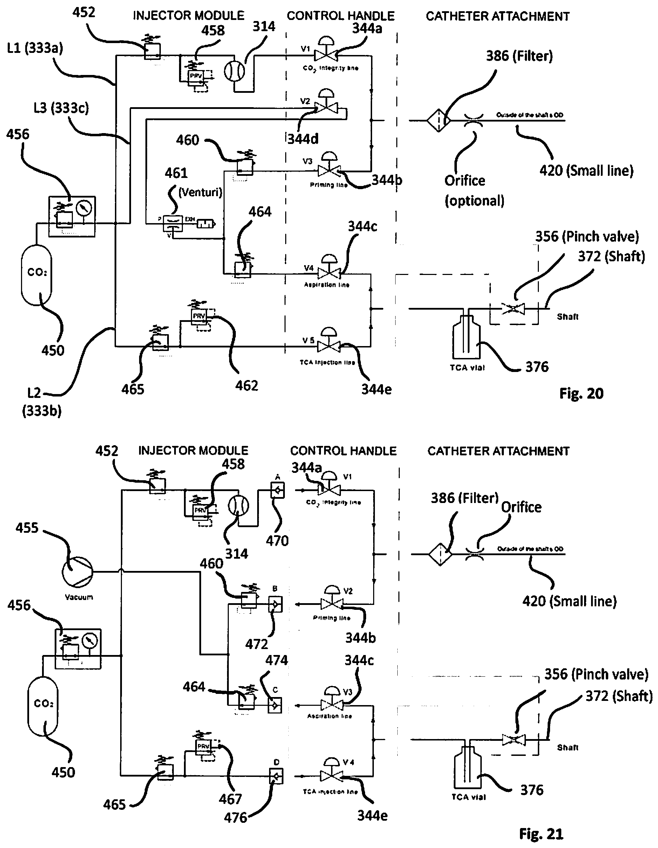

Use of the system 300 will now be described in conjunction with Table 1 below and the flow diagram of Figure 20. Note the Slider 1 in Table 1 below corresponds to actuator 334 and the Slider 2 corresponds to actuator 336. The valves V1-V5 in Table 1 and Figure 20 correspond to valves 344a - 344e of Figures 11A and 11B as follows:

344a » V1
344b » V3
344c » V4
344d » V2
344e » V5

Table 1.

| | | | Trumpet valves | | | | | Pinch valve |
|---|---|---|---|---|---|---|---|---|
| State | Slider 1 position | Slider 2 position | V1 $CO_2$ | V2 Venturi | V3 Priming | V4 Suction | V5 TCA injection | TCA |
| Setup | 0 | 0 | - | - | - | - | - | - |
| OFF | 0 | 0 | - | - | - | - | - | - |
| Integrity test | 1 | 0 | + | - | - | - | - | - |
| OFF | 0 | 0 | - | - | - | - | - | - |
| TCA fill | 0 | 2 | - | + | + | | + | + |
| OFF/Treatment | 0 | 0 | - | - | - | | - | - |
| Empty | 0 | 3 | + | + | - | + | - | + |

As shown in the above Table 1, in the initial Setup mode (State), slider 1 is in the rearward position and slider 2 is in the neutral position (within slots 339 and 337, respectively, of Figure 9). In this initial position of the sliders 1 and 2, all five trumpet valves V1-V5 and the pinch valve (pin 362 moved by lever 356 of Figure 12) are closed. In the OFF mode (state) the sliders 1 and 2 and valves V1-V5 are in the same position as in the Setup mode.

In the first step of the procedure, to conduct the cavity integrity test to detect perforations as described above, slider 1 is moved forward to advance cam plate 338 of Figure 11A into engagement with rear camming surface of cam cap 348a to open valve V1 to enable $CO_2$ flow. Valves V2-V5 and the pinch valve remain closed. After the cavity integrity test, when it is confirmed there is no leakage, slider 1 is returned to its initial proximal position to retract cam plate 338 to enable cap 348a to return to its upper position to close valve V1 to stop $CO_2$ flow. At this point, at the off mode (state), all the valves are in the closed position.

Next, the agent injection mode is initiated. Slider 1 remains in its rearward (proximal) position and slider 2 is moved from its neutral (middle) position to the advanced position to open valve V5 and the pinch valve to enable flow of TCA (or other agent as described herein). Note as slider 2 moves forward, cam plate 342 moves forward so that pin 356b rides within groove 358 of Figures 11A and 11B to move pin 362 away from post 366 to unpinch the tube 416 (Figure 14) to open the pinch valve. Such forward movement of the cam plate 42 also causes engagement of the rear camming surface of cap 349e to open valve 344e (V5). Additionally, forward movement of slider 2 also opens valve V2 for the venturi and valve V3 for priming as rear camming surface of cap 348d of valve 344d and of cap 348b of valve 344b, are engaged by cam plate 342. After the agent is injected and fills the cavity, the slider 2 is returned (retracted) to its initial neutral position to retract cam plate 342 to close valves V5, V2, V3 and the pinch valve. The treatment state commences as the agent is left in the cavity for tissue ablation. The valves V1, V2, V3, V4, V5 and the pinch valve remain closed during the treatment mode (State). After a predetermined time in the treatment mode, e.g., five minutes (although other time periods are also contemplated for sufficient tissue penetration and treatment), the slider 2 is retracted back from its neutral position to retract cam plate 342 to open valve 1 for $CO_2$ flow, valve V2 for the venturi, valve V4 for suction and the pinch valve. Valves V3 (priming) and V5 (injection) suction remain closed.

Turning now to the flow diagram of Figure 20, the system functions and components will now be explained in more detail. During the system setup and preparation for use of this embodiment, the pinch valve and all trumpet valves preferably remain closed. In alternate embodiments, one or more valves can have a normally open position and are moved to the closed position by the user for setup and preparation.

First, the integrity test of the cavity is initiated. The $CO_2$ cartridge 450, mounted/placed within or adjacent the injector module 312, supplies $CO_2$ for the integrity check and pressurized agent injection. A pressure regulator 456 for the $CO_2$ is provided. The pressure regulator 456 provides for the initial/primary reduction of the pressure inside of the $CO_2$ cartridge 450, e.g., from 2,000-3,000 psi to 20-50 psi. Other regulators (secondary) provide for further reduction and control of pressure to the levels prescribed for the integrity check and pressurized agent injection, e.g., from 20-50 psi to less than one (1) psi. When the slider 334 is moved forward, the camming surface of plate 346 opens valve V1 (valve 344a) so $CO_2$ travels through the tubing 394 and into the small tube in the shaft of the catheter as represented by Line L1 (333a). That is, the uterine cavity is insufflated with $CO_2$ inflow from $CO_2$ source 450 as the gas flows through line L1. A pressure regulator 452 and flow meter 314 regulate and monitor flow and pressure. Pressure relief valve 458 provides a safety feature. When the cavity is filled and it is confirmed there are no perforations, the flow meter 314 shows that the flow has stopped. That is, when the pressure inside of the cavity equalizes with the pressure set by the regulator 452, the flow of $CO_2$ stops as indicated by the flow meter 314. The cessation can be automatic or alternatively or in addition by the user monitoring the pressure and stopping flow manually. The slider 334 is then returned to its initial position which moves cam plate 346 to shut off valve V1 to cut off $CO_2$ flow.

Upon completion of the integrity test, with the trumpet valve V1 (344a) closed, the agent injection mode is then initiated. Slider 336 is slid forward to open valve V2 (344d) see line L3 (333c) for the venturi (461) and open valve V3 (344b) to open the priming line (priming subsystem) which is attached to the small tube within the catheter shaft 372 as suction is applied to the line activating the priming subsystem (priming mode) that creates negative pressure in the lines and the cavity. Simultaneous with opening of valves V2 and V3, agent injection valve V5 (344e) and the pinch valve are open so pressurized agent can be injected from the vial 376 into the lumen of the catheter shaft 372. Note a negative pressure regulator 460 for the priming line is provided to control negative pressure level in the priming subsystem. This is beneficial for minimizing the amount of the agent entering the line 420. Alternatively, instead of an active negative pressure priming with the venturi pump 461, the valve V2 could passively vent/purge air and gas from the lines and the cavity. Also note Line 2 (333b) for agent injection includes pressure regulator 465 and pressure relief valve 462. As the agent fills the cavity, the pressure inside of the cavity is rising. Once the pressure equalizes with the pressure set by the pressure regulator 465, the flow of the agent stops. After the agent fills the cavity, the slider 336 is moved back to the neutral position to close the valves and the agent is left in the cavity for a period of time. At this point, all valves are shut and there is no pressure.

In some embodiments, the agent containing vial has graduation markings as shown in Figure 17A. The markings on vial 376' could indicate the volume of the agent being injected and expressed in volume units, e.g., milliliters. The volume of the uterine cavity is measured, for example by ultrasonography, prior to treatment. The user visually monitors the

agent flow using graduation markings as an indicator to assure that the injected volume from the vial does not exceed the pre-measured volume of the cavity. Thus, if the clinician observes an excess volume of agent flowing out of the vial, the clinician can stop the injection. In alternate embodiments, the system can automatically stop injection before an excess volume is delivered.

In alternate embodiments, instead of measuring the uterine cavity volume, the length of the uterine cavity is measured, for example with a uterine sound device or ultrasonography (dimension "L" on Fig. 17). The length of the cavity could serve as an approximation of the uterine cavity volume. In such embodiments, the markings are expressed in length units, e.g., centimeters, and correspond to the dimension "L" (Fig. 17A).

A marking A1 shows the level of the agent A in the vial prior to treatment. When the agent injection is initiated as described above, the agent first fills the shaft 370 before flowing into the cavity via the openings 372. A marking A2 shows the level of the agent after the shaft 370 is filled, but before the agent flows into the cavity. At that point zero (0) cm of agent is injected into the cavity. The marking A3 shows the approximate volume of the agent that is expected to be injected into the cavity with the length of 4 cm. Other indication scales and markings of the vial are also contemplated.

As an example, if the length of the uterine cavity L=4cm, the marking A3 shows the approximate volume of the agent that is expected to be injected into the cavity. If the flow of the agent does not stop automatically (as described above) prior to the agent volume dropping to the marking A3, the user can stop the flow manually by returning the slider 336 to the neutral position.

The therapeutic agent remains in the cavity for the duration of the treatment. At the end of the treatment, e.g., after a preset time, the cavity and the lines are emptied by sliding actuator 336 rearward opening valve V2 and the aspiration system by opening valve V4 activating the venturi while optionally simultaneously opening (activating) valve V1 that reopens flow of $CO_2$. The pinch valve is also opened. This allows the aspirated therapeutic agent to flow into the vial 376. Once the flow of the agent from the cavity and the lines ends, the actuator is returned to its neutral position and valves V1, V2 and V4, and the pinch valve, are closed. Note the pressure regulator of the aspiration subsystem is designated by reference numeral 464.

As can be appreciated, after the set treatment time, valve V4 for aspiration and valve V1 for $CO_2$ flow are simultaneously opened. Suction is applied to create a vacuum to pull agent back into the vial 376. Opening valve V1 allows $CO_2$ back through the small line tube 420 to prevent the uterine cavity from collapsing under the negative aspiration pressure and facilitate the agent flow from the cavity back to the vial 376 (reintroducing $CO_2$). Note when applying vacuum, some agent might get into the small catheter tube 420, however, if the $CO_2$ is activated, it will blow the agent out of the tube and prevent the cavity from collapsing under negative pressure.

Note that the system of Figure 20 provides a venturi pump instead of an external vacuum. The venturi pump is powered by the same $CO_2$ cartridge 450 and primary regulator 456. The system could still leak $CO_2$, so to preserve $CO_2$, a valve is provided, i.e., valve V1, to cut off $CO_2$ flow so the valve V1 is only open when $CO_2$ is needed. In this manner, the venturi is not open all the time.

In an alternate embodiment, instead of a venturi, an external suction source is provided. This system is depicted in Table 2 below and the flow diagram of Figure 21.

Table 2

| | | | Trumpet valves | | | | Pinch valve |
|---|---|---|---|---|---|---|---|
| State | Slider 1 position | Slider 2 position | V1 $CO_2$ | V2 Priming | V3 Suction | V4 TCA injection | TCA |
| Setup | 0 | 0 | - | - | - | - | - |
| OFF | 0 | 0 | - | - | - | - | - |
| Integrity test | 1 | 0 | + | - | - | - | - |
| OFF | 0 | 0 | - | - | - | - | - |
| TCA fill | 0 | 2 | - | + | | + | + |
| OFF/Treatment | 0 | 0 | - | - | | - | - |
| Empty | 0 | 3 | + | - | + | - | + |

Table 2 is the same as Table 1 except that it does not have a venturi pump so there are four valves instead of five. The system otherwise operates in the same way with the valves opening in response to movement of slider 1 (slider 334) and slider 2 (slider 336) and thus for brevity is not repeated herein as the aforedescribed functions of the slider 1. slider 2 and activation of valves for $CO_2$, printing, suction and agent injection in Table 1 are fully applicable to this embodiment of

Table 2. The flow diagram of Figure 21 differs from the diagram of Figure 20 in the absence of the venturi and venturi valve V2 and in the provision of an external suction source 455 in line with the priming subsystem and aspiration subsystem. It also differs in the provision of check valves 470, 472, 474, and 476 for valves V1-V4, respectively. All other components of the system/subsystems are the same and therefore are labeled with the same reference numerals as in Figure 20 and for brevity are not further described since the discussion of these systems in conjunction with Table 1 and Figure 20 are fully applicable to the systems/subsystems of Figure 21 (except for the venturi of Figure 20).

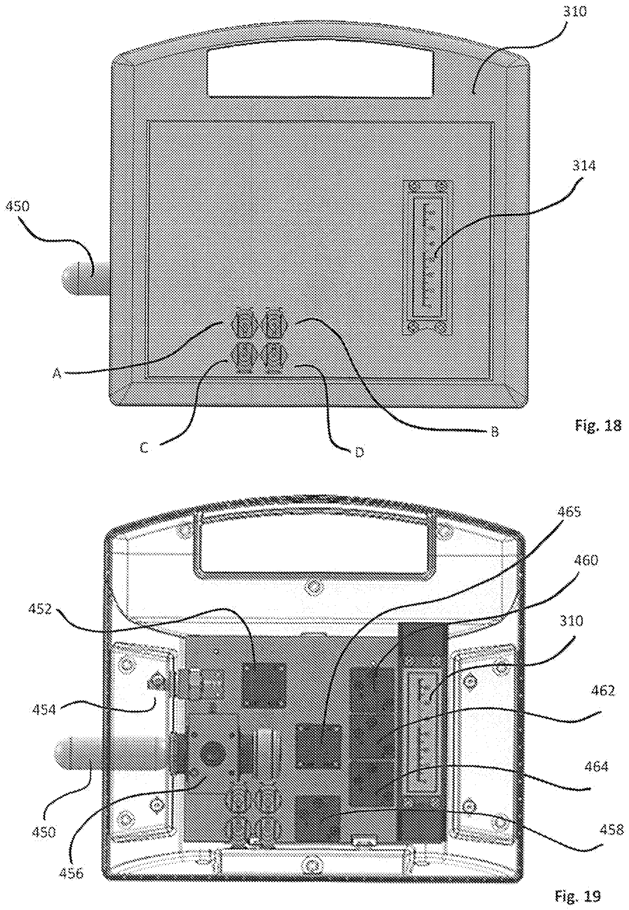

Note that Figures 18 and 19 show one arrangement of the regulators, relief valves, etc. of the system in the injection module which labeled components can be understood by reference to Figure 20. Other arrangements/positions are also contemplated.

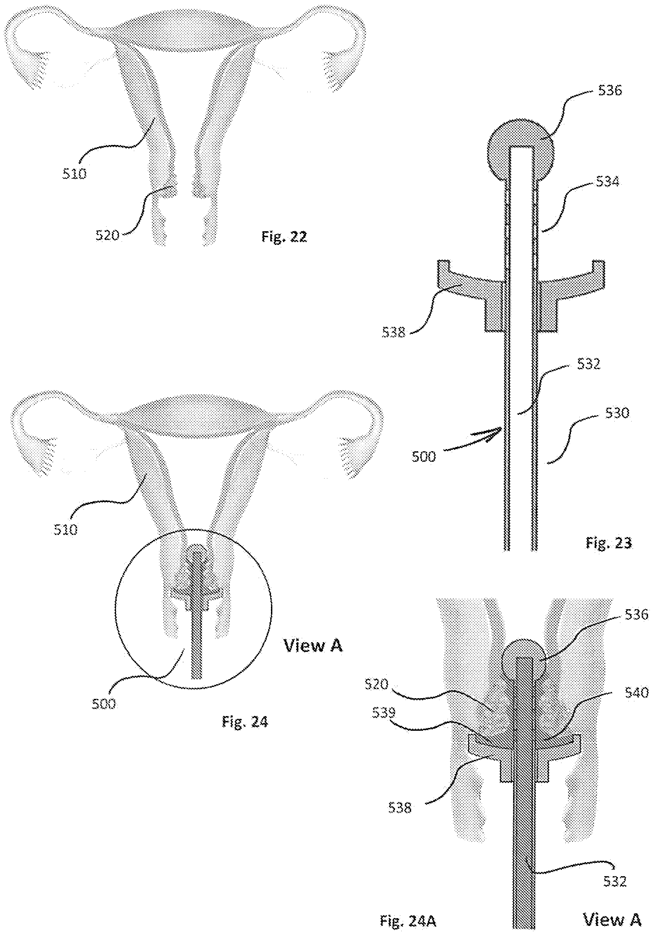

In addition to treatment of heavy menstrual bleeding that is described in detail above, the ablation agent could be used for treatment of other conditions, for example vaginal and cervical lesions that are caused by human papilloma virus (HPV). In some preferred embodiments, the ablation agent has viscosity higher than water. One example of such lesions is cervical intraepithelial neoplasia (CIN) grades 1-3. Fig. 22 shows CIN 520 on the cervical portion of the uterus 510.

Fig. 23 shows a delivery system 500 for application of the ablation agent 540 to the cervical surface and/or the cervical canal. The system 500 has a shaft 530 with a lumen 532 and at least one side opening 534. The ablation agent 540 is injected into the lumen 532 using an injection system similar to the systems described above or another pressurization device, such as a syringe. Unlike the intrauterine injection application described above that require precise pressure control, control of pressure in this clinical application is not as critical. The ablation agent 540 travels via the lumen 532 and exits through at least one opening 534 in shaft 530. The opening(s) 534 is preferably located in the distal portion of the shaft 530. After the system 500 is inserted into the cervical canal (Fig. 24-24A), a cervical plug 536 seals the canal to prevent flow of the ablation agent distally to the shaft 530. A cervical cap 538 distributes the agent 540 over the target tissue and prevents leakage of the agent into the vaginal cavity. Concave surface 539 of cap 538 helps direct agent flow to the side as the cavity is sealed between cervical plug 536 and cervical cap 538. Once the agent is injected, it will remain in contact with tissue for the duration of the treatment, then evacuated using the aspiration subsystem described above or suctioned by a syringe. Alternatively, it could be applied and left on tissue until it is absorbed or metabolized.

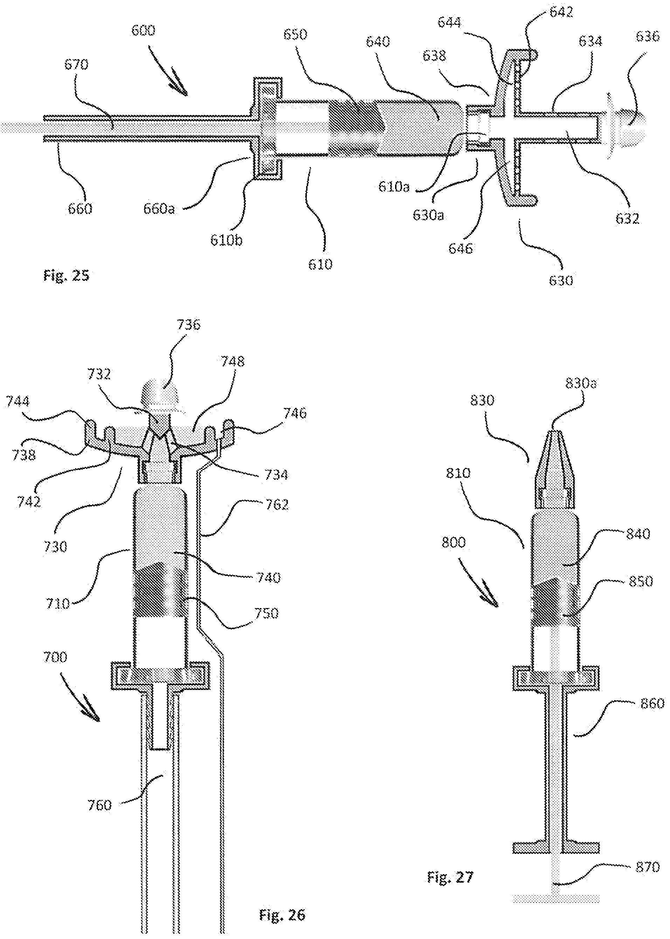

Similar to the delivery system 500, Fig. 25 shows a delivery system 600 for application of the ablation agent 640 to the cervix and/or the cervical canal. Other uses for the system 600 are also contemplated. The system 600 has a delivery tip 630 and a syringe-style cartridge 610, preferably made of borosilicate glass, with a cartridge plunger 650, preferably made from a Teflon-coated elastomer. The cartridge 610 is preferably prefilled with the agent 640 to avoid transferring the agent into a different container at the treatment location, such as a doctor's office. This cartridge 610 with the integrated cartridge plunger 650 and a stopper plug (not shown) is suitable for storage and transportation of the agent 640 during its shelf life. The cartridge 610 can have a flange 610a at a distal region for attachment of the tip 630 using a neck 630a as it engages as recess in the neck 630a as shown. The cartridge 610 can also have a flange 610b at a proximal region for attachment of a cartridge holder 660 with a flange adapter 660a. As shown, the flange 610b is captured within the adapter 660a of the cartridge holder 660. The cartridge holder 660 extends proximally and connects to a control handle similar to other injection systems described above. In some embodiments, the plunger 650 is moved with a mechanical actuator 670 that is operated by the control handle.

The cartridge 610 is preferably connected/attached to the tip 630 right before use. Alternatively, the cartridge 610 could be attached to the tip 630 during manufacture. The delivery tip 630 has a lumen 632 with at least one side opening 634 communicating with the lumen and a cervical cap 638. A radial wall 642 and cervical cap 638 form an internal space 646 that communicates with the lumen 632 and the cartridge 610. The radial wall 642 has at least one opening 644, and preferably a plurality of openings.

The ablation agent 640 is injected out of the cartridge 610 into the delivery tip 630 by moving the plunger 650 forward distally. In some embodiments, if the cartridge 610 is supplied with a stopper plug, the neck 630a could be equipped with a tubular spike to pierce through the stopper to allow the agent 640 to flow into the delivery tip 630. The agent 640 flows into the internal space 646 and the lumen 632 of delivery tip 630. The agent 640 is distributed over the target tissue when it exits the delivery tip 630 via the openings 634 and 644. A cervical plug 636 prevents the agent 640 from flowing deeper into the cervical canal as it blocks distal flow beyond the target tissue. In some embodiments, after the treatment is complete, the plunger 650 is retracted aspirating the ablation agent 640 back through the tip 630 and into the cartridge 610. The plunger is preferably operated manually but it is also contemplated that in alternate embodiments the plunger can be pneumatically actuated.

A system 700 shown on Fig. 26 is similar to the system 600, but it is preferably used for treatment of exocervix with no or limited exposure of the cervical canal. For this reason, an axial extension 732 of an applicator 730 is short and has no side openings in the distal portion for the agent flow into the cervical canal in contrast to side openings 634 of tip 630 of system 600. Extension 732 has at least one proximal side opening 734 near a cervical cap 738 for the agent 740 flow from a cartridge 710 into the applicator 730. Extension 732 is used for centering/stabilizing of the system 700 relative to the cervix and plugging it with a cervical plug 736. A cervical plug 736 prevents the agent 740 from flowing distally in the cervical canal as it blocks distal flow beyond the target tissue. A circumferential wall 742 and cervical cap 738 form external space 748. In

some embodiments, the ablation agent 740 has viscosity that would allow the external space 748 to be prefilled with the agent 740 prior to the deployment of the system 700. A circumferential wall 744, the circumferential wall 742 and the cervical cap 738 form a suction chamber 746. In some embodiments, the applicator 730 is made from a rigid material, while in other embodiments it is made out of flexible elastomeric material. In some other embodiments only a portion of the applicator is made from an elastomer, for example the circumferential wall 744. A suction line 762 is connected to the suction chamber 746.

Unlike the system 600, the plunger 750 is operated pneumatically using a line 760 that connects the system 700 to a $CO_2$ pressurized system described above. Alternatively, the plunger can be operated manually. The line 762 is connected to the aspiration/suction of that system. Once the system 700 is inserted into the body, and the applicator 730 is pressed against tissue, the suction is applied via the line 762. In a preferred embodiment, a vacuum created in the suction chamber 734 pulls the tissue creating a seal that prevents leakage of the agent 740. Otherwise, the user can just press the system 700 against the tissue forming a seal.

A system 800 shown in Fig. 27 is suitable for topical applications of the agent 840, which is preferably in the gel form. A cartridge holder 860 has a plunger pusher 870 that moves the plunger 850. When the pusher 870 is advanced distally by a user, the plunger 850 injects the agent 840 through a distal aperture 830a of a nozzle 830, which is preferably detachable. Multiple configurations of the nozzle 830 and shapes/sizes of the aperture 830a are contemplated depending on the clinical application and viscosity of the ablation agent.

The foregoing systems 600, 700, and 800 provide examples of the clinical applications of the therapeutic agents disclosed herein. These agents preferably have a high viscosity to better control flow to the target tissue while reducing flow to healthy tissue, thereby protecting healthy tissue from unwanted ablation or tissue damage. Flow is controlled not only by the cervical plug in some of these embodiments but also by the composition of the agent. Various formulations of the agent and thickener are described below. As can be appreciated, these formulations are also applicable to the endometrial ablation devices described herein.

In some embodiments, injection of the agent can be visually observed via ultrasound. This can enable the clinician to monitor flow to target tissue and observe unwanted flow to healthy tissue. In some embodiments, ultrasound can be used instead of a cavity integrity check as the clinician can visually observe via agent flow presence/absence of perforations.

## Therapeutic agent

The therapeutic agents of the present teaching are used for treatment of tissue inside of the body, including treatment of various body cavities. In some embodiments, the therapeutic agent is used for ablation of endometrial and myometrial layers of the uterine cavity in human female patients, for example, to treat abnormal uterine bleeding. The therapeutic agent can be delivered/injected/instilled into the uterine cavity utilizing a system that is described herein. Alternatively, it can used with a system described in commonly U.S. Patent No. 10,485,962, and/or U.S. publication 2020/0261707. In addition to uterine cavity ablation, the therapeutic agent can be used in a variety of clinical applications and applied to the body using the various devices/systems disclosed herein. For example, ablation of cervical and vaginal lesions, anogenital warts, skin warts and other conditions caused by HPV. It should also be appreciated that the unique formulations of the agent containing compositions described in detail below can also be applied utilizing apparatus other than those disclosed herein.

Various substances/compositions are described herein. The apparatus is designed in preferred embodiments to deliver the therapeutic agent in the form of a viscous chemical agent (substance) for a chemical endometrial ablation procedure.

The therapeutic agent can be delivered into the uterine cavity without direct visualization or with visualization using common imaging techniques.

The therapeutic agent in some embodiments can contain trichloroacetic acid in an aqueous solution as an active ingredient. Concentration of trichloroacetic acid above 50% is preferred, although a lower concentration could also be sufficient. Other active ingredients, such as other acids, that are suitable for tissue treatment, such as tissue ablation, are also contemplated.

Unique anatomy of fallopian tubes naturally restricts flow from the uterine cavity to prevent vaginal infections from spreading to abdomen. Gynecologists inject the uterine cavity with $CO_2$ gas and saline for various diagnostic procedures. Many of these procedures require intentional perfusion of fallopian tubes. It is well-established that no flow of these low-viscosity fluids could occur in the tubes unless the intrauterine pressure exceeds 60 mmHg for $CO_2$ and 70 mmHg for saline. Therefore, an ablation/therapeutic agent with viscosity that exceeds viscosity of $CO_2$ and saline is beneficial for the safety of the proposed treatment.

The delivery systems of the present invention for delivery of the agent have a number of other safety features. One feature is the cavity integrity test that allows the user to detect any possible undiagnosed perforations or potential leakage path via fallopian tubes or the cervical canal prior to injection of the therapeutic/ablation agent. The test is done by

insufflating the cavity with $CO_2$ gas. Another safety feature is the injection/instillation of the therapeutic agent at the pressure that is equal or below the pressure level that is used for the cavity integrity test. One of the reasons that the $CO_2$ gas is selected as a medium for the test is that it has a viscosity of 0.0147 centipoise at 20 degrees of Celsius, which is significantly lower than the viscosity of trichloroacetic acid that is used as a therapeutic agent (approximately 1 centipoise or higher). So, if the low-viscosity $CO_2$ gas doesn't leak out of the uterine cavity at the insufflation pressure, then the therapeutic agent with a higher viscosity cannot leak out either when injected at an equal or lower pressure. Increasing the difference in viscosity between $CO_2$ and the therapeutic agent will further enhance safety. It should also be appreciated that with a therapeutic agent of sufficiently increased viscosity, the pressure level of the agent's injection could be above 70mmHG, i.e., above the pressure level of 60mmHG of the $CO_2$ for the cavity integrity test, which is below that what would cause tubal leak yet still sufficient to detect perforation. This is due to the inherently limited flow of the agent because of its increased viscosity.

The therapeutic agent could be in a liquid or a gel form with viscosity ranging for example from about 0.1 (one tenth) to about 100,000 (one hundred thousand) centipoises, but preferably between about 1 (one) and about 30,000 (thirty thousand) centipoises. The high viscosity avoids accidental leakage, but the viscosity level needs to be balanced so its viscosity is not too high that, in some applications that require injection, it can't flow under pressure. Preferably, the viscosity also needs to be balanced to allow the agent to flow within the cavity from the area where it is injected to other areas of the cavity to assure that the cavity is fully filled without moving the catheter relative to the cavity.

To maximize safety and effectiveness of the procedure, a high viscosity therapeutic agent is injected at a pressure level that would not cause its perfusion into fallopian tubes, however would fully fill the target body cavity or lumen. In the endometrial ablation application, it is also important that the diameter of the shaft of the delivery device is sized to avoid a need for the cervical canal dilation in most of the cases. Therefore, the preferred viscosity of the agent is balanced for the preferred injection pressure of below about 60 mmHg (but could alternately be higher depending on the viscosity as mentioned above) and the preferred diameter of the catheter shaft lumen less than about 6mm. Larger shaft lumen diameters are also contemplated. Preferably, the length of the shaft for the devices intended for cervical and uterine treatments should allow users to operate these devices from outside of the body. The length of the shaft is optimized/minimized to allow the agent to flow at the lowest pressure possible. That is, a longer length shaft will result in/require a higher pressure. The preferred length of the device is about 20 cm, however longer and shorter lengths are also contemplated. Similarly to the necessary balance between the injection pressure and viscosity, aspiration pressure should also be balanced with the agent's viscosity to assure that the agent could be evacuated, and that the cavity is sufficiently emptied. All this needs to be balanced with the amount of time that is required to fill and empty the cavity.

In some embodiments, where the therapeutic agent is used for topical application, for example ablation of Cervical Intraepithelial Neoplasia (CIN) that are caused by HPV, it would be beneficial to provide a high viscosity therapeutic agent to the exocervix (or "ectocervix") and, optionally, to a proximal portion of the cervical canal, which might also be affected by the virus. This could be accomplished with an applicator, such as a delivery device described above. In such embodiments, the viscosity should be balanced such that the agent's viscosity allows the agent to flow and fill the space that is formed between the target tissue and the device to assure that the targeted tissue is fully exposed to the agent, while healthy tissue is preserved as the agent flow is designed to be limited to the target tissue. The viscosity is also balanced so it can be readily held and then released from the applicator if applied by a topical applicator. As explained herein, the high viscosity is achieved by a composition containing the agent and a thickener. Such composition in preferred embodiments has a high concentration of the agent (highly acidic; low pH) which enhances tissue ablation and enhances the tissue penetrative capabilities as discussed herein.

Yet, in some other embodiments, where the therapeutic agent is used for topical application, for example ablation of skin warts, genital warts, various nail disorders and other tissue treatments that require tissue ablation/distraction, it could be applied directly to the target tissue with an applicator, such as a delivery device described above or a spatula, with or without use of a pressurized injection system. In such embodiments, the viscosity is preferably balanced, such that the agent's viscosity is adequate for remaining on the target tissue for the duration of the treatment and a) not too low, so that it could be easily applied to the target tissue without risk of spreading/leaking onto the adjacent tissue that does not require treatment, and b) not too high, such that it would not adhere to an applicator and pull away with it.

Regardless of the viscosity level, high-viscosity agents described above are in a fluid state that would allow the agent to conform to the target surface. This is especially beneficial in treatment of lesions, such as pre-cancerous or cancer, where any omitted/untreated area of the target presents a clinical risk.

The therapeutic agent could be a Newtonian or non-Newtonian fluid. In non-Newtonian fluids, viscosity can change under force. So, when a non-Newtonian fluid is pressurized for injection, its viscosity could be lower or higher than when the pressurizing force is removed. Preferably, the viscosity of the therapeutic agent is lower while it is being injected, so that it can flow better through the lines from the vial to the body cavity. Once the cavity is filled, and the injection stopped, the viscosity of the therapeutic agent increases when it stays inside of the cavity for the duration of the treatment. It is contemplated that certain formulations of the therapeutic agent would allow it to stay in the cavity until it is metabolized instead of being evacuated at the end of the procedure.

One option for a non-Newtonian formulation could be based on use of a pseudoplastic semi-liquid delivery system utilizing microencapsulation technology.

The viscosity of the therapeutic agent could be increased by using various thickeners, such as a cross-linked polymer, such as Carbopol manufactured by Lubrizol Advanced Materials, Inc., or Natrosol hydroxyethylcellulose manufactured by Ashland. Other thickeners could include polysaccharide, polysiloxanes, natural rubber, glycerin, petrolatum, paraffin, lanolin, beeswax, rosin, Xanthan gum, amylopectine, cellulose, carboxymethyl cellulose or similar materials that could be suitable for pharmaceutical applications.

Use of polymers for changing viscosity of acidic aqueous compositions having active agents such as TCA include products within the Carbopol® polymer family such as crosslinked polyacrylic acid polymers that are utilized in pharmaceutical products as rheology modifiers. Carbopol® polymers are used to develop semisolid and liquid formulations with a wide range of flow and rheological properties. Carbopol® polymers are highly efficient thickeners, suspending agents and stabilizers at low usage levels on the weight-by-weight (w/w) basis (0.1 - 3.0% w/w). Performance of Carbopol depends on the pH level of the formulation with maximum viscosity typically achieved at a pH of 6.0 - 7.0. For example, Lubrizol (Lubrizol Corporation) reports that a solution that has 0.2% of Carbopol 971p NF has the viscosity of appr. 300 cP at pH 3, however the viscosity with the same amount of Carbopol 971p NF reaches appr. 2,500 cP with at pH 7. By comparison, when the amount of Carbopol 971p NF is increased to 2% w/w, the viscosity is appr. 4,400 cP at pH 3 and 14,400 cP at pH 7. Thus, an increase in polymer concentration results in an increase in viscosity, therefore Lubrizol recommends use of less than 3% w/w for solutions and suspensions, topical preparations, and even oral solids for immediate release. Usage of higher level of polymer is only recommended for oral solid dose for extended release. Lubrizol makes similar recommendations with regards to the polymer content for other Carbopol thickeners.

In some embodiments of the present description the thickener, such as Carbopol, is over 3% w/w of the composition. When thickeners, such as Carbopol, are used in the amount over 3% w/w, the composition becomes too viscous making it practically impossible to mix in other ingredients. for example, TCA crystals, if the thickener is first added to/mixed with water or another solvent, as it is typically done. Thus, in some embodiments, the composition (substance) could be formulated by first mixing water with the TCA in concentration above 50% w/w, then adding a thickener. Low pH level that results from such high concentration of TCA reduces Carbopol's ability to increase viscosity of liquids. In some embodiments with pH less than 2, and more preferably less than 0 (zero), with the Carbopol content of 3.01 to about 4 w/w%, range, the viscosity of the agent is up to about 3,000 cP (three thousand) ("syrup-like" viscosity), which is below of the viscosity reported by Lubrizol with less than 2% w/w of Carbopol. Such high-viscosity agents could be used in the applications similar to HMB described above. As explained above, to keep the injection pressure of the solution for treatment of HMB below 60 mmHg and the shaft diameter less than 6 mm, the viscosity of up to 3,000 cP is preferred, however a higher viscosity is also contemplated. (Note that other percentages of thickener for the compositions of the present teaching are also contemplated).

In some other embodiments, at a similar pH level, a thickener could be used in the amount between about 4% and about 5% w/w achieving viscosity of the agent up to 10,000 cP (ten thousand) (higher viscosity is also contemplated), which is a "honey-like" or "molasses-like" viscosity. Such high-viscosity agents could be used in the applications similar to ablation of CIN lesions described above.

In some other embodiments, at a similar pH level, a thickener could be used in the amount over 5% w/w resulting in viscosity between 10,000 (ten thousand) and 100,000 cP (one hundred thousand). Such high-viscosity agents, with high concentration of agent, e.g., over 50% w/w of agent such as TCA, could be used in the topical applications, such as skin and nail conditions mentioned above.

Because TCA crystals are dissolved in water or another appropriate liquid before the thickener is added, the agent preparation presents no processing challenges.

It should be appreciated that percentages of TCA, water, and thickener can differ depending on desired substance and/or desired clinical applications.

Human Papillomavirus virus (HPV), such as warts, verrucae, etc. only grows in the epidermis, the layer of the skin closest to the surface. Other skin conditions, such as corns and calluses, actinic keratoses also form on the epidermis. Epidermis is only 0.05 mm thick around eyelids, but can be 1.5 mm thick at the palms of the hands or the soles of the feet. Conditions, such as acne, affect the skin's middle layer - the dermis, which has a thickness of 1.2 mm in forehead and cheeks. Another condition caused by HPV is Cervical Intraepithelial Neoplasia (CIN) that affects squamous epithelium. The squamous epithelium is 0.5 mm thick and has 10-20 layers, including an outer superficial layer. These conditions if treated by an ablation agent that is for example a substance (composition) that contains a low concentration of the agent (e.g., TCA) and pH of around or above 2 would lack sufficient tissue penetrative capabilities and only treat it superficially.

The inventors of the current application conceived that deeper penetration of tissue can achieve clinically efficacious results and conceived that this could be achieved with higher concentration of TCA with lower pH level. The higher concentration is necessary for the TCA to achieve the required tissue penetration. For example, CIN lesions could affect tissue as deep as 4.8mm, and, therefore, an effective treatment should ablate tissue to 5-7 mm depth. Thus, in one embodiment of the present description, the composition contains over 50% w/w TCA (and preferably over 60% w/w TCA),

a thickener of over 3% w/w and a pH of less than 2, e.g., one or zero. The pH range in some embodiments can be between about -2 (negative two) and below about 2 (positive two). In an alternate embodiment, the composition has negative pH, i.e., below 0 (zero), Such high concentration of TCA with low pH and high viscosity penetrates tissue to a depth greater than 1 mm, and preferably in 3-7mm range or even higher. Thus, the formulations of the treatment agent of the present teaching optimize the balance of TCA concentration for tissue penetration and thickness to control flow. In embodiments of the TCA used for high pressure injection, such balance of high TCA concentration and thickener is also balanced with the ability to enable high pressure injection (not too solid because if too viscous it cannot be injected under pressure). The formulation is created so the high viscosity TCA can be used for endometrial ablation as described herein, as well as other applications such as treatment of HPV or cancerous cervix lesions. Thus, methods of the present teaching include the agent formulations described herein used in such treatments. Various combinations of all devices and methods described above may be utilized in the same procedure, sequentially and/or simultaneously.

It should be appreciated that in some embodiments, compositions comprising less than 50% weight by weight of trichloroacetic agent and less than 3% weight by weight are also contemplated for ablation of tissue beyond a superficial level. While such compositions are not as effective, deeper tissue penetration could be achieved by increasing treatment time and repeat.

Although the systems, devices, apparatus and methods of the subject teaching have been described with respect to preferred embodiments, those skilled in the art will readily appreciate that changes and modifications may be made thereto.

The present invention is set out in the claims that follow.

## Claims

1. A composition for ablation of tissue beyond a superficial level, the composition comprising at least 3% weight by weight of a thickening agent to increase the viscosity of the composition and at least 50% weight by weight of trichloroacetic acid to lower pH of the composition to less than 2, wherein the composition is configured to penetrate the tissue beyond the superficial layer while applying the composition to target tissue during application without spreading to healthy tissue.

2. The composition of claim 1, wherein the pH is less than or equal to zero.

3. The composition of claim 1 or claim 2, wherein the thickening agent is Carbopol.

4. A device for ablation of tissue beyond a superficial level in a cervical canal, the device comprising: a composition according to claim 1, 2 or 3, said composition containing a therapeutic agent to be applied beyond the target tissue;

   a seal to seal the cervical canal to block flow of the composition; and
   a lumen for injecting the composition over the target tissue in the cervical canal, wherein, in use, the therapeutic agent is configured to be maintained in contact with target tissue for a period of time to enable penetration of the therapeutic agent beyond the superficial level and beyond a depth of 1mm.

5. The device of claim 4, further comprising a cap to laterally spread the composition within the cervical canal.

6. A composition for use in the treatment of tissue beyond a superficial level, the composition containing a therapeutic agent, the composition having at least 3% weight by weight of a thickening agent to provide a viscosity greater than a viscosity of water, over 50% weight by weight of trichloroacetic acid to provide a pH less than 2, thereby enabling, in use, the therapeutic agent to remain on the tissue for a predetermined period of time to penetrate the tissue beyond the superficial layer.

7. The composition for use as claimed in claim 6, wherein the agent is configured to remain on tissue without spreading to adjacent non-targeted tissue due to the viscosity of the agent.

8. The composition for use as claimed in claim 6, wherein the agent is applied via a pressurized injection system.

9. The composition for use as claimed in claim 6, wherein the agent treats conditions caused by HPV.

10. The composition for use as claimed in claim 6, wherein the agent is configured to penetrate more than 1mm, but less than 10mm.

**11.** The composition for use as claimed in claim 6, wherein the composition is in the form of a gel.

**12.** The composition for use as claimed in claim 6, wherein the superficial layer is selected from one of the group consisting of (i) uterine endometrium; (ii) epithelium of the cervix; (iii) Exocervix and (iv) epidermis around eyelids.

**13.** The composition for use as claimed in claim 6, wherein the viscosity exceeds a viscosity of $CO_2$ and saline.

**14.** The composition of use as claimed in claim 6, wherein the agent is configured to be applied with a topical applicator.

**15.** The composition of use as claimed in claim 6, wherein the viscosity is 1 (one) to 30.000 (thirty thousand) centipoises.

**Patentansprüche**

**1.** Zusammensetzung zur Ablation von Gewebe über eine oberflächliche Ebene hinaus, wobei die Zusammensetzung Folgendes umfasst: mindestens 3 Gewichts-%, bezogen auf das Gewicht, eines Verdickungsmittels, um die Viskosität der Zusammensetzung zu erhöhen, und mindestens 50 Gewichts-%, bezogen auf das Gewicht, Trichloressigsäure, um den pH der Zusammensetzung auf kleiner als 2 zu senken, wobei die Zusammensetzung zur Durchdringung des Gewebes über die oberflächliche Ebene hinaus konfiguriert ist, während die Zusammensetzung während der Anwendung auf das Zielgewebe ohne Verteilung auf gesundes Gewebe angewendet wird.

**2.** Zusammensetzung nach Anspruch 1, wobei der pH kleiner als oder gleich null ist.

**3.** Zusammensetzung nach Anspruch 1 oder Anspruch 2, wobei das Verdickungsmittel Carbopol ist.

**4.** Vorrichtung zur Ablation von Gewebe über eine oberflächliche Ebene in einem Zervikalkanal hinaus, wobei die Vorrichtung Folgendes umfasst: eine Zusammensetzung nach Anspruch 1, 2 oder 3, wobei die Zusammensetzung ein Therapeutikum enthält, das über das Zielgewebe hinaus anzuwenden ist;

eine Dichtung, um den Zervikalkanal abzudichten, um das Fließen der Zusammensetzung zu blockieren;
ein Lumen zum Einspritzen der Zusammensetzung über das Zielgewebe in dem Zervikalkanal, wobei das Therapeutikum bei Verwendung konfiguriert ist, um mit dem Zielgewebe für eine Zeitdauer im Kontakt zu bleiben, um die Durchdringung des Therapeutikums über die oberflächliche Ebene hinaus und über eine Tiefe von 1 mm hinaus zu ermöglichen.

**5.** Vorrichtung nach Anspruch 4, die weiter eine Kappe umfasst, um die Zusammensetzung innerhalb des Zervikalkanals lateral zu verteilen.

**6.** Zusammensetzung zur Verwendung bei der Behandlung von Gewebe über eine oberflächliche Ebene hinaus, wobei die Zusammensetzung ein Therapeutikum enthält, wobei die Zusammensetzung Folgendes aufweist: mindestens 3 Gewichts-%, bezogen auf das Gewicht, eines Verdickungsmittels, um eine Viskosität bereitzustellen, die größer ist als eine Viskosität von Wasser, über 50 Gewichts-%, bezogen auf das Gewicht, Trichloressigsäure, um einen pH bereitzustellen, der kleiner als 2 ist, wodurch ermöglicht wird, dass das Therapeutikum bei Verwendung auf dem Gewebe für eine vorgegebene Zeitdauer verbleibt, um das Gewebe über die oberflächliche Ebene hinaus zu durchdringen.

**7.** Zusammensetzung zur Verwendung nach Anspruch 6, wobei das Mittel konfiguriert ist, um auf Gewebe zu verbleiben, ohne auf benachbartes nicht-anvisiertes Gewebe aufgrund der Viskosität des Mittels verteilt zu werden.

**8.** Zusammensetzung zur Verwendung nach Anspruch 6, wobei das Mittel über ein unter Druck gesetztes Einspritzsystem angewendet wird.

**9.** Zusammensetzung zur Verwendung nach Anspruch 6, wobei das Mittel Leiden behandelt, die durch HPV verursacht werden.

**10.** Zusammensetzung zur Verwendung nach Anspruch 6, wobei das Mittel konfiguriert ist, um mehr als 1 mm, doch weniger als 10 mm zu durchdringen.

**11.** Zusammensetzung zur Verwendung nach Anspruch 6, wobei die Zusammensetzung in der Form eines Gels vorliegt.

**12.** Zusammensetzung zur Verwendung nach Anspruch 6, wobei die oberflächliche Schicht aus einer der Gruppe ausgewählt ist, die aus Folgenden besteht: (i) Gebärmutterschleimhaut; (ii) Epithel der Zervix; (iii) Exozervix und (iv) Epidermis um Augenlider.

**13.** Zusammensetzung zur Verwendung nach Anspruch 6, wobei die Viskosität eine Viskosität von $CO_2$ und Kochsalzlösung übersteigt.

**14.** Zusammensetzung zur Verwendung nach Anspruch 6, wobei das Mittel konfiguriert ist, um mit einem topischen Applikator angewendet zu werden.

**15.** Zusammensetzung zur Verwendung nach Anspruch 6, wobei die Viskosität 1 (eins) bis 30 000 (dreißigtausend) Centipoise beträgt.

**Revendications**

**1.** Composition pour l'ablation de tissu au-delà d'un niveau superficiel, la composition comprenant au moins 3 % en poids par poids d'un agent épaississant pour augmenter la viscosité de la composition et au moins 50 % en poids par poids d'acide trichloroacétique pour faire baisser le pH de la composition à moins de 2, la composition étant configurée pour pénétrer le tissu au-delà de la couche superficielle lors de son application à un tissu cible sans étalement jusqu'à un tissu sain.

**2.** Composition selon la revendication 1, dans laquelle le pH est inférieur ou égal à zéro.

**3.** Composition selon la revendication 1 ou la revendication 2, dans lequel l'agent épaississant est le Carbopol.

**4.** Dispositif pour l'ablation de tissu au-delà d'un niveau superficiel dans un canal cervical, le dispositif comprenant : une composition selon la revendication 1, 2 ou 3, ladite composition contenant un agent thérapeutique destiné à être appliqué au-delà du tissu cible ;

un sceau pour sceller le canal cervical afin de bloquer l'écoulement de la composition ; et
une lumière pour injecter la composition par-dessus le tissu cible dans le canal cervical, dans lequel, lors de l'utilisation, l'agent thérapeutique est configuré pour rester en contact avec le tissu cible pendant une période de temps afin de permettre la pénétration de l'agent thérapeutique au-delà du niveau superficiel et au-delà d'une profondeur de 1 mm.

**5.** Dispositif selon la revendication 4, comprenant en outre une coiffe pour étaler latéralement la composition à l'intérieur du canal cervical.

**6.** Composition destinée à être utilisée dans le traitement d'un tissu au-delà d'un niveau superficiel, la composition contenant un agent thérapeutique, la composition ayant au moins 3 % en poids par poids d'un agent épaississant pour fournir une viscosité supérieure à celle de l'eau, plus de 50 % en poids par poids d'acide trichloroacétique pour fournir un pH inférieur à 2, permettant ainsi à l'agent thérapeutique, en cours d'utilisation, de rester sur le tissu pendant une période de temps prédéterminée pour pénétrer dans le tissu au-delà de la couche superficielle.

**7.** Composition destinée à être utilisée selon la revendication 6, dans laquelle l'agent est configuré pour rester sur le tissu sans s'étaler jusqu'à un tissu non ciblé adjacent en raison de la viscosité de l'agent.

**8.** Composition destinée à être utilisée selon la revendication 6, dans laquelle l'agent est appliqué au moyen d'un système d'injection sous pression.

**9.** Composition destinée à être utilisée selon la revendication 6, dans laquelle l'agent traite des conditions causées par le HPV.

**10.** Composition destinée à être utilisée selon la revendication 6, dans laquelle l'agent est configuré pour pénétrer sur plus de 1 mm, mais moins de 10 mm.

**11.** Composition destinée à être utilisée selon la revendication 6, la composition se présentant sous la forme d'un gel.

**12.** Composition destinée à être utilisée selon la revendication 6, dans laquelle la couche superficielle est sélectionnée parmi l'un des tissus du groupe comprenant (i) l'endomètre utérin ; (ii) l'épithélium du col de l'utérus ; (iii) l'exocervix et (iv) l'épiderme autour des paupières.

**13.** Composition destinée à être utilisée selon la revendication 6, dans laquelle la viscosité dépasse une viscosité de $CO_2$ et de sérum physiologique.

**14.** Composition destinée à être utilisée selon la revendication 6, dans laquelle l'agent est configuré pour être appliqué avec un applicateur topique.

**15.** Composition destinée à être utilisée selon la revendication 6, dans laquelle la viscosité est 1 (un) à 30 000 (trente mille) centipoises.

1
10
30
32
28
26
19
18
20
14
38
24
36
16
12
22
25
34

Fig. 1

14
16b
15
12
16
19
18
40
42
44
12a
16a
46
46a
46b

Fig. 2

54
48a
56a
52
62
48
58
19
56
18

Fig. 3

INJECTOR MODULE

CONTROL HANDLE

CATHETER ATTACHMENT

112 114 116 118 V1 $CO_2$ integrity line

V2 128 70 62 56

122 124 126 V3 130 Priming line 34 64 56a

104 106 V4 138

$CO_2$ 102

132 134 136 32 Activation line 38 24 66 72 18 Waste Stopcock Shaft

142 144 28 Injection line 36 22 68 Agent vial

Fig. 4

60a
204a
90
206
204
208a
60
212
200
208
60b
220

Fig.5

200
222
230
208
212
228
224
226
204
206

Fig. 6

INJECTOR MODULE
CONTROL HANDLE
CATHETER ATTACHMENT
300
V1
$CO_2$ integrity line
V2
PRV
Integrity / Sensor tube
208
V3
Sensor line
316
V4
$CO_2$
P
V
Aspiration line
Shaft
204
TCA injection line
222

Fig. 7

300
312
314
310
374
372a
376
330a
331
333
372b
372
373
371
330
370
376
330
338
337
336
377
378
374'
372c
335
332
339
334
372a
373
374a
372b
370

Fig. 8

Fig. 9

377
378
338
334
344b
344a
342
332
330
342a
336
344e
346
344c
344d
351e
346
348e
349e
348b
348d
348a
351c
348c
349c
344e
344b
344d
344a
344c
342a
348b
358a
342
358b
358
358c
344b
358
352
358b
354
356c
356
356a
366
332
373a
373
362
364
330u
356b
330a

Fig. 10

Fig.11A

Fig. 11B

Fig. 12

376
382
384
378
374'
372
376
386
392
382
388
384
376
392a
392
388
389
386
396
384
377
398
394a
394
402
416
388
394c
406
382
404
420
386
414
384
372
396
408
402a
410
394a
394
394b

Fig. 13A

Fig. 13B

Fig. 13C

Fig. 14

376
383
418
404
372
420
375
362
416
385
330
420
375
422
372e
422
375a
372c
372
421
372b
426
372d
372b
L
374a
374'
372a
376'
A1
A2
A
A3
0
2
4
6 cm
Fig. 15

Fig. 16

Fig. 17

Fig. 17A

310
314
450
A
B
C
D
Fig. 18
465
460
452
310
454
462
464
450
458
456
Fig. 19

452
INJECTOR MODULE
CONTROL HANDLE
CATHETER ATTACHMENT
458
314
344a
L1 (333a)
V1
$CO_2$ integrity line
386 (Filter)
L3 (333c)
V2
344d
456
460
Outside of the shaft's OD
V3
461
(Venturi)
Priming line
344b
Orifice
(optional)
420 (Small line)
464
344c
356 (Pinch valve)
372 (Shaft)
V4
Aspiration line
$CO_2$
PRV
Shaft
450
V5
TCA injection line
TCA vial
376
L2
(333b)
465
462
344e
Fig. 20
452
INJECTOR MODULE
458
CONTROL HANDLE
CATHETER ATTACHMENT
344a
V1
$CO_2$ integrity line
386 (Filter)
Orifice
455
470
Vacuum
314
460
V2
Priming line
420 (Small line)
456
344b
472
474
344c
356 (Pinch valve)
464
V3
Aspiration line
372 (Shaft)
Shaft
$CO_2$
450
V4
TCA injection line
376
TCA vial
465
467
476
344e
Fig. 21

510
520
536
534
538
532
530
500
510
500
View A
536
520
540
539
538
532
View A

Fig. 22

Fig. 23

Fig. 24

Fig. 24A

600
670
650
640
644
642
638
634
636
660
660a
610b
610
610a
630a
646
632
630
736
732
748
744
746
738
734
742
730
762
740
710
750
700
760
830a
830
810
840
800
850
860
870

Fig. 25

Fig. 26

Fig. 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10485962 B
- US 20190381294 A
- US 20200261707 A
- US 2005255039 A